(19) Europäisches Patentamt
European Patent Office
Office européen des brevets

(11) EP 4 162 924 A2

(12) EUROPÄISCHE PATENTANMELDUNG

(43) Veröffentlichungstag:
**12.04.2023 Patentblatt 2023/15**

(21) Anmeldenummer: **22192610.8**

(22) Anmeldetag: **29.08.2022**

(51) Internationale Patentklassifikation (IPC):
***A61K 8/58*** *(2006.01)* ***A61Q 5/06*** *(2006.01)*

(52) Gemeinsame Patentklassifikation (CPC):
**A61Q 5/065; A61K 8/585;** A61K 2800/805

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **07.10.2021 DE 102021211314**

(71) Anmelder: **Henkel AG & Co. KGaA**
**40589 Düsseldorf (DE)**

(72) Erfinder:
- **Jaiser, Phillip**
  **40764 Langenfeld (DE)**
- **Lechner, Torsten**
  **40764 Langenfeld (DE)**
- **Schoepgens, Juergen**
  **41366 Schwalmtal (DE)**
- **Nowottny, Marc**
  **41069 Mönchengladbach (DE)**
- **Mathiaszyk, Carsten**
  **45277 Essen (DE)**
- **Tairi, Avni**
  **47138 Duisburg (DE)**
- **Kruppa, Carolin**
  **40721 Hilden (DE)**
- **Walter, Andreas**
  **40880 Ratingen (DE)**

# (54) KOSMETISCHE ZUSAMMENSETZUNG, DIE DURCH DAS VERMISCHEN VON ZWEI SILAN-BLENDS ERHALTEN WIRD

(57) Gegenstand der vorliegenden Anmeldung ist ein Verfahren zur Herstellung einer kosmetischen Zusammensetzung zur Behandlung von keratinischem Material, insbesondere menschlichen Haaren, die erhalten wird durch Vermischen von einem ersten Mittel (A) mit einem zweiten Mittel (B), wobei
- das erste Mittel (A) erhalten wird durch Reaktion von einem oder mehreren organischen $C_1$-$C_6$-Alkoxysilanen (a1) mit Wasser (a2),
wobei das oder die organischen $C_1$-$C_6$-Alkoxysilane (a1) ausgewählt sind aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)triethoxysilan, (3-Dimethylaminopropyl)tri-methoxysilan, (2-Dimethylaminoethyl)triethoxysilan und (2-Dimethyl-aminoethyl)-trimethoxysilan, und
- das zweite Mittel (B) erhalten wird durch Reaktion von einem oder mehreren organischen $C_1$-$C_6$-Alkoxysilanen (b1) mit Wasser (b2),
wobei das oder die organischen $C_1$-$C_6$-Alkoxysilane (b1) ausgewählt sind aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Vinyltrimethoxysilan, Vinyltriethoxysilan Tetramethoxysilan und Tetraethoxysilan,
dadurch gekennzeichnet, dass

- das Mittel (A) keine organischen $C_1$-$C_6$-Alkoxysilane aus der Gruppe (b1) enthält und
- das Mittel (B) keine organischen $C_1$-$C_6$-Alkoxysilane aus der Gruppe (a1) enthält.

Die Anmeldung betrifft auch eine kosmetische Zusammensetzung, die über dieses Herstellungsverfahren erhalten wird, sowie die Verwendung dieser Zusammensetzung zur Behandlung keratinischem Material, insbesondere zur Färbung davon.

EP 4 162 924 A2

## Beschreibung

Die vorliegende Anmeldung betrifft ein Verfahren zur Herstellung einer kosmetischen Zusammensetzung, die durch Vermischen der beiden Mittel (A) und (B) erhalten wird. Bei den beiden Mitteln (A) und (B) handelt es sich um zwei verschiedeneSilan-Blends. Das erste Mittel (A) wird erhalten durch Reaktion von einem oder mehreren reaktiven Silanen vom Typ der Aminoalkyl-$C_1$-$C_6$-Alkoxysilane (a1) mit Wasser (a2). Das zweite Mittel (B) wird erhalten durch Reaktion von einem oder mehreren reaktiven Silanen vom Typ der Alkyl-$C_1$-$C_6$-Alkoxysilane (b1) mit Wasser (b2). Kennzeichnend für beide Mittel ist, dass das Mittel (A) keine organischen $C_1$-$C_6$-Alkoxysilane aus der Gruppe (b1) enthält, und dass das Mittel (B) keine organischen $C_1$-$C_6$-Alkoxysilane aus der Gruppe (a1) enthält.

Ein zweiter Gegenstand der vorliegenden Erfindung ist eine kosmetische Zusammensetzung, die über das Herstellverfahren des ersten Erfindungsgegenstands erhalten wurde.

Ein dritter Gegenstand ist die Verwendung einer kosmetischen Zusammensetzung, die über das Herstellverfahren des ersten Erfindungsgegenstands erhalten wurde, zur Behandlung keratinischem Material, insbesondere zur Färbung von keratinischem Material, insbesondere zur Färbung von menschlichen Haaren.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Schamponierungen besonders widerstandsfähig sind.

Bei den in der EP 2168633 B1 eingesetzten organischen Silicium-Verbindungen handelt es sich um reaktive Verbindungen aus der Klasse der Alkoxy-Silane. Diese Alkoxy-Silane hydrolysieren in Gegenwart von Wasser mit hoher Geschwindigkeit und bilden - abhängig von den jeweils eingesetzten Mengen an Alkoxy-Silan und Wasser - Hydrolyseprodukte und/oder Kondensationsprodukte aus. Der Einfluss der in dieser Reaktion eingesetzten Wassermenge auf die Eigenschaften des Hydrolyse- bzw. Kondensationsproduktes werden beispielsweise in WO 2013068979 A2 beschrieben.

Wenn diese Hydrolyse- bzw. Kondensationsprodukte auf keratinischem Material angewendet werden, bildet sich auf dem Keratinmaterial ein Film oder auch ein Coating aus, welches das Keratinmaterial vollständig umhüllt und auf diese Weise die Eigenschaften des Keratinmaterials stark beeinflusst. Mögliche Anwendungsbereiche sind beispielsweise das permanente Styling oder auch die permanente Formveränderung von Keratinfasern. Hierbei werden die Keratinfasern mechanisch in die gewünschte Form gebracht und in dieser Form dann durch Ausbildung des vorbeschriebenen Coatings fixiert. Eine weitere ganz besonders gut geeignete Anwendungsmöglichkeit ist die Färbung von Keratinmaterial; im Rahmen dieser Anwendung wird das Coating bzw. der Film in Gegenwart einer farbgebenden Verbindung, zum Beispiel eines Pigments, erzeugt. Der durch das Pigment gefärbte Film verbleibt auf dem Keratinmaterial bzw. den Keratinfasern, und es resultieren überraschend waschbeständige Färbungen.

Der große Vorteil des auf Alkoxy-Silanen basierenden Färbeprinzips liegt darin, dass die hohe Reaktivität dieser Verbindungsklasse ein sehr schnelles Coating ermöglicht. So können bereits nach sehr kurzen Anwendungszeiträumen von nur wenigen Minuten extrem gute Färbeergebnisse erzielt werden. Neben diesen Vorteilen birgt die hohe Reaktivität der Alkoxy-Silane jedoch auch einige Nachteile. So können bereits geringfügige Änderungen der Produktions- und Anwendungsbedingungen, wie beispielsweise die Änderung von Luftfeuchtigkeit und/oder Temperatur, zu starken Schwankungen der Produktleistung führen. Vor allem haben die zu dieser Erfindung führenden Arbeiten gezeigt, dass die Alkoxy-Silane extrem sensibel auf die Bedingungen reagieren, die bei der Herstellung der Keratinbehandlungsmittel herrschen.

Weiterführende analytische Untersuchungen haben gezeigt, dass bei der Herstellung verschiedener Silan- bzw. Siloxan-Gemische und Blends komplexe Hydrolyse- und Kondensations-Reaktionen ablaufen, die abhängig von den gewählten Reaktionsbedingungen zu oligomeren Produkten unterschiedlicher Molekülgröße und verschiedenen Vernetzungsgrades und führen. In diesem Zusammenhang hat sich herausgestellt, dass das Molekulargewicht dieser Silan-Oligomere großen Einfluss auf die späteren Produkteigenschaften nehmen kann. Werden bei der Herstellung die falschen Bedingungen gewählt, so kann dies zur Ausbildung von zu großen oder zu kleinen Silan-Kondensaten führen, wodurch die spätere Produktleistung, insbesondere das spätere Färbevermögen auf dem Keratinmaterial, negativ beeinflusst wird.

Es war die Aufgabe der vorliegenden Anmeldung, optimierte Mittel und Verfahren für die Behandlung von menschlichen Haaren aufzufinden. Die in diesen Mittel bzw. Verfahren eingesetzten Gemische aus Alkoxy-Siloxanen sollten auf gezielte Weise so hergestellt werden, dass bei einer anschließenden Anwendung die optimalen anwendungstechnischen Eigenschaften erzielt werden können. Die auf diese Weise hergestellten Mittel sollten einen idealen Vernetzungsgrad bzw. Siloxan-Oligomere mit optimaler Verteilung des Molekulargewichts besitzen, wodurch eine Verbesserung der Färbeleistung erzielt werden soll. In diesem Zusammenhang war es ganz besonders wichtig, die Herstellung der Mittel reproduzierbar zu gestalten. Auf diesem Wege sollten die Mittel bei ihrer Anwendung in einem Färbeverfahren zu Färbungen mit höherer Farbintensität und verbesserten Echtheitseigenschaften unter Standardisierung des Farbergebnisses führen. Insbesondere die Waschechtheit und die Reibechtheit sollten auf reproduzierbare Weise verbessert werden. Weiterhin sollten die auf diesem Wege hergestellten Mittel besonders lagerstabil sein, und das spätere färberische Potential der Mittel sollte nicht von ihrer Lagerzeit abhängen.

Überraschenderweise hat sich nun herausgestellt, dass die vorgenannte Aufgabe hervorragend gelöst werden kann, wenn auf dem Keratinmaterial kosmetische Zusammensetzungen zur Anwendung kommen, die vor der Anwendung durch Vermischen von zwei Mitteln (A) und (B) hergestellt werden. Die beiden Mittel (A) und (B) stellen verschiedene Silan-Blends dar, wobei in jedem der beiden Mittel $C_1$-$C_6$-Alkoxy-Silane einer bestimmten Gruppe getrennt voneinander hydrolysiert und oligomerisiert werden. Für die beiden Hydrolyse-Reaktionen können in beiden Mitteln (A) und (B) jeweils spezifische Wassermengen eingessetzt werden.

Ein erster Gegenstand der vorliegenden Erfindung ist ein Verfahren zur Herstellung einer kosmetischen Zusammensetzung zur Behandlung von keratinischem Material, insbesondere menschlichen Haaren, die erhalten wird durch Vermischen von einem ersten Mittel (A) mit einem zweiten Mittel (B), wobei

- das erste Mittel (A) erhalten wird durch Reaktion von einem oder mehreren organischen $C_1$-$C_6$-Alkoxysilanen (a1) mit Wasser (a2),
  wobei das oder die organischen $C_1$-$C_6$-Alkoxysilane (a1) ausgewählt sind aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)triethoxysilan, (3-Dimethylaminopropyl)tri-methoxysilan, (2-Dimethylaminoethyl)triethoxysilan und (2-Dimethyl-aminoethyl)-trimethoxysilan, und
- das zweite Mittel (B) erhalten wird durch Reaktion von einem oder mehreren organischen $C_1$-$C_6$-Alkoxysilanen (b1) mit Wasser (b2),

  wobei das oder die organischen $C_1$-$C_6$-Alkoxysilane (b1) ausgewählt sind aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Vinyltrimethoxysilan, Vinyltriethoxysilan Tetramethoxysilan und Tetraethoxysilan,
  dadurch gekennzeichnet, dass

- das Mittel (A) keine organischen $C_1$-$C_6$-Alkoxysilane aus der Gruppe (b1) enthält und
- das Mittel (B) keine organischen $C_1$-$C_6$-Alkoxysilane aus der Gruppe (a1) enthält.

Verkürzt ausgedrückt ist der erste Gegenstand ein Verfahren zur Herstellung einer kosmetischen Zusammensetzung, die erhalten wird durch Vermischen von einem ersten Mittel (A) mit einem zweiten Mittel (B), wobei

- das erste Mittel (A) erhalten wird durch Reaktion von einem oder mehreren organischen $C_1$-$C_6$-Alkoxysilanen (a1) mit Wasser (a2),
  wobei das oder die organischen $C_1$-$C_6$-Alkoxysilane (a1) ausgewählt sind aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)triethoxysilan, (3-Dimethylaminopropyl)tri-methoxysilan, (2-Dimethylaminoethyl)triethoxysilan und (2-Dimethyl-aminoethyl)-trimethoxysilan, und
- das zweite Mittel (B) erhalten wird durch Reaktion von einem oder mehreren organischen $C_1$-$C_6$-Alkoxysilanen (b1) mit Wasser (b2),

wobei das oder die organischen $C_1$-$C_6$-Alkoxysilane (b1) ausgewählt sind aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Vinyltrimethoxysilan, Vinyltriethoxysilan Tetramethoxysilan und Tetraethoxysilan,
dadurch gekennzeichnet, dass

- das Mittel (A) keine organischen $C_1$-$C_6$-Alkoxysilane aus der Gruppe (b1) enthält und
- das Mittel (B) keine organischen $C_1$-$C_6$-Alkoxysilane aus der Gruppe (a1) enthält.

Das erste Mittel (A) wird erhalten wird durch Reaktion von einem oder mehreren organischen $C_1$-$C_6$-Alkoxysilanen (a1) mit Wasser (a2), wobei diese $C_1$-$C_6$-Alkoxysilane (a1) vom Typ der Aminoalkyl-$C_1$-$C_6$-alkoxysilane sind.

Das zweite Mittel (B) wird erhalten wird durch Reaktion von einem oder mehreren organischen $C_1$-$C_6$-Alkoxysilanen (b1) mit Wasser (b2), wobei diese $C_1$-$C_6$-Alkoxysilane (b1) vom Typ der Alkyl-Ci-$C_6$-alkoxysilane sind.

Überraschenderweise konnte gefunden werden, dass es durch die getrennte Hydrolyse und Kondensation der beiden Silan-Typen in den beiden Mitteln (A) und (B) möglich wird, die Reaktionen der beiden unterschiedlich reaktiven Silan-Typen viel besser zu steuern. Nach Vermischen der beiden Mittel (A) und (B) konnte auf diesem Wege eine kosmetische Zusammen- erhalten werden, die bei Anwendung auf dem Haar besonders gute anwendungstechnische Eigenschaften erzielt. Überraschend war vor allem, dass diese kosmetische Zusammensetzung im Vergleich zu den über andere Methoden hergestellten Zusammensetzungen eine besonders gute Lagerstabilität besaß und dass sich die mit dieser Zusammensetzung erhaltenen Ergebnisse, insbesondere Farbergebnisse, ganz besonders gut reproduzieren ließen.

Bei der späteren Anwendung auf dem Haar bilden die auf diese Weise hergestellten kosmetischen Zusammensetzungen besonders stabile und reproduzierbare Filme bzw. Coatings aus, deren Eigenschaften unabhängig von der Anwendungsdauer der Zusammensetzung sind. Weiterhin hat sich gezeigt, dass die kosmetischen Zusammensetzungen, welche durch Vermischen der beiden Mittel (A) und (B) hergestellt wurden, bei Einsatz in einem Färbeprozess zu sehr intensiven und gleichmäßigen Färbungen mit sehr guter Deckkraft, Reibechtheit und Waschechtheit führten.

keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Kosmetische Zusammensetzung zur Behandlung von keratinischem Material

Unter einer kosmetischen Zusammensetzung zur Behandlung von keratinischem Material wird beispielsweise ein Mittel zur Färbung des Keratinmaterials, ein Mittel zur Umformung oder Formgebung von keratinischem Material, insbesondere keratinischen Fasern, oder auch ein Mittel zur Konditionierung bzw. zur Pflege des keratinischen Materials verstanden. Besonders gute Eignung zeigen die über das erfindungsgemäße Verfahren hergestellten kosmetischen Zusammensetzungen zur Färbung von keratinischem Material, insbesondere zur Färbung von keratinischen Fasern, bei denen es sich besonders bevorzugt um menschliche Haare handelt.

Der Begriff "kosmetische Zusammensetzung zur Färbung" wird im Rahmen dieser Erfindung für eine durch Einsatz von farbgebenden Verbindungen, wie beispielsweise Pigmenten, Mica, thermochromen und photochromen Farbstoffen, direktziehenden Farbstoffen und/oder Oxidationsfarbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich *in situ* durch Oligomerisierung bzw. Kondensation des oder der organischen Siliciumverbindungen, wobei die farbgebende(n) Verbindung(en) mit diesem Film bzw. diesem Coating wechselwirken und darin eingelagert bzw. von diesem umschlossen werden.

kosmetischen Zusammensetzung

Die kosmetische Zusammensetzung des ersten Erfindungsgegenstandes wird durch Vermischen der beiden Mittel (A) und (B) erhalten. Für das Vermischen können die beiden Mittel (A) und (B) beispielsweise miteinander verrührt, verschüttelt oder auch auf andere Art miteinander vermengt werden. Zum Zweck des Vermischens kann zum Beispiel das Mittel (A) vorgelegt und das Mittel (B) hinzugegeben werden, oder aber das Mittel (B) wird vorgelegt und im Anschluss

daran das Mittel (A) hinzugefügt.

Die aus dem Vermischen der beiden Mittel (A) und (B) resulierende kosmetische Zusammen-setzung kann vor der Anwendung auf dem Keratinmaterial noch für einen bestimmten Zeitraum von einigen Tagen, Wochen oder Monaten gelagert werden. Dies kann beispielsweise der Fall sein, wenn die kosmetische Zusammensetzung verpackt und dem Anwender oder Friseur als Teil eines kosmetischen Produktes oder einer Mehrkomponenten-Verpackungseinheit für die Behandlung des Keratinmaterials zur Verfügung gestellt wird.

Ebenfalls ist es möglich, die aus dem Vermischen der beiden Mittel (A) und (B) resulierende kosmetische Zusammensetzung im Anschluss an das Vermischen zeitnah auf das keratinische Meterial aufzutragen.

Weiterhin ist es ebenfalls möglich, die aus dem Vermischen der beiden Mittel (A) und (B) resulierende kosmetische Zusammensetzung vor dem Auftragen auf das keratinische Material zusätzlich noch mit einer oder mehreren weiteren kosmetischen Zusammensetzungen zu vermischen.

Schließlich ist es ebenfalls möglich und erfindungsgemäß, wenn die nach dem Vermischen der Mittel (A) und (B) erhaltene kosmetische Zusammensetzung noch eine weitere Reaktion durchläuft, bei der beispielsweise eine fortgesetzte Hydrolyse, Oligomerisierung und/oder Kondensation stattfinden kann, die mit oder Einsatz eines Katalysators durchgeführt wird. Diese Ausführungsform ist ganz besonders bevorzugt.

Mittel (A)

Das das erste Mittel (A) wird erhalten wird durch Reaktion von einem oder mehreren organischen $C_1$-$C_6$-Alkoxysilanen (a1) mit Wasser (a2).

Organische $C_1$-$C_6$-Alkoxy-Silane (a1) im Mittel (A)

Bei dem oder den organischen $C_1$-$C_6$-Alkoxy-Silanen (a1) handelt es sich um organische, nicht polymere Siliciumverbindungen, die aus der Gruppe aus (3-Aminopropyl)triethoxysilan, (3-Amino-propyl)trimethoxysilan, (2-Aminoethyl)triethoxysilan, (2-Aminoethyl)trimethoxysilan, (3-Dimethylaminopropyl)triethoxysilan, (3-Dimethylaminopropyl)trimethoxysilan, (2-Dimethylaminoethyl)triethoxysilan und (2-Dimethylaminoethyl)trimethoxysilan ausgewählt sind.

Die zur Lösung der erfindungsgemäßen Aufgabenstellung besonders gut geeigneten organischen $C_1$-$C_6$-Alkoxy-Silanen (a1) sind

- (3-Aminopropyl)triethoxysilan

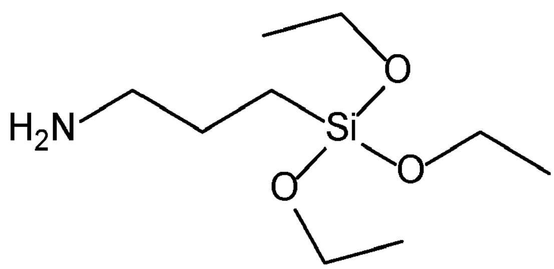

- (3-Aminopropyl)trimethoxysilan

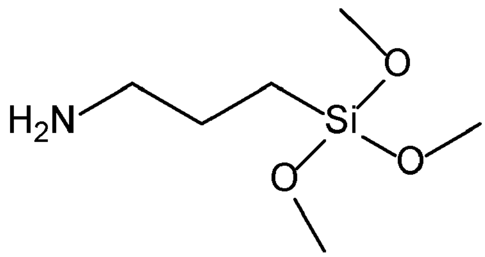

- (2-Aminoethyl)triethoxysilan

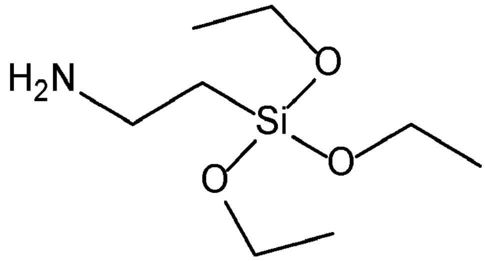

- (2-Aminoethyl)trimethoxysilan

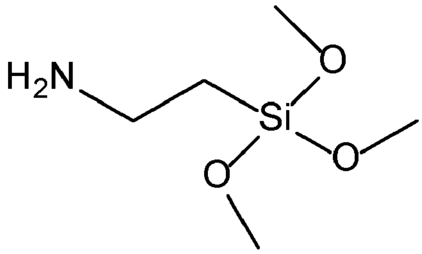

- (3-Dimethylaminopropyl)triethoxysilan

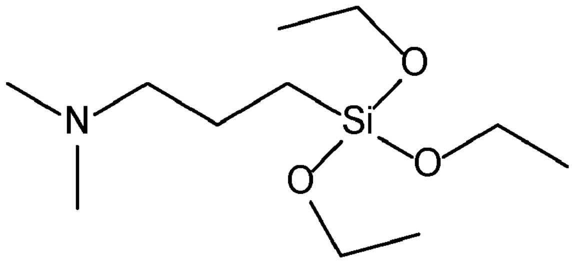

- (3-Dimethylaminopropyl)trimethoxysilan

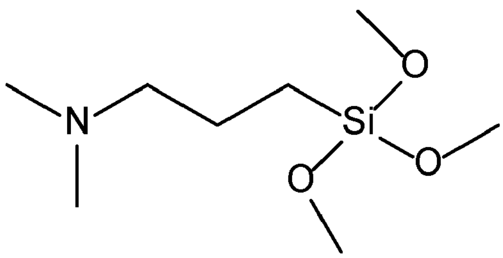

- (2-Dimethylaminoethyl)triethoxysilan.

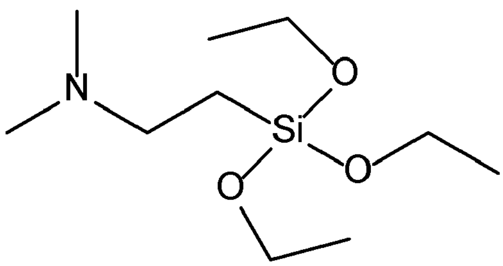

- (2-Dimethylaminoethyl)trimethoxysilan und/oder

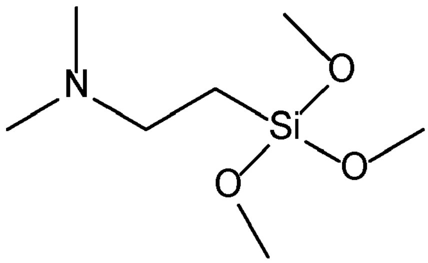

Die vorgenannten organische Siliciumverbindungen (a1) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Bei der Herstellung des ersten Mittels (A) durch werden eine oder mehrere organische $C_1$-$C_6$-Alkoxysilane (a1) mit Wasser (a2) zur Reaktion gebracht. Besonders gute Ergebnisse wurden erhalten, wenn als organisches $C_1$-$C_6$-Alkoxysilan (a1) (3-Aminopropyl)triethoxysilan eingesetzt wurde. Am allermeisten bevorzugt ist es daher, wenn das erste Mittel (A) erhalten wird durch Reaktion von 3-Aminopropyl)tri-ethoxysilan (a1) mit Wasser (a2).

gezielte Hydrolyse der organische $C_1$-$C_6$-Alkoxysilane (a1) im Mittel (A) durch Zugabe von Wasser

Bei der Herstellung des Mittels (A) werden ein oder mehrere organische $C_1$-$C_6$-Alkoxysilane (a1) mit Wasser (a2) versetzt, um eine gezielte Hydrolyse und hierdurch bedingt eine Vorkondensation in Gang zu setzen.

Da im Mittel (A) ausschließlich Aminoalkyl-$C_1$-$C_6$-alkoxysilane miteinander oligomerisieren, wird im Mittel (A) ein Oligomer gebildet, welches ausschließlich durch gezielte Hydrolyse und Kondensa-tion der Aminoalkyl-$C_1$-$C_6$-alkoxysilane ausgebildet wird. Damit enthalten alle Monomerbestandteile des im Mittel (A) vorhandenen Oligomers bzw. Reaktionsproduktes eine Aminoalkyl-Gruppe.

Die Reaktion mit dem Wasser (a2) kann beispielsweise durch Zutropfen oder Zugießen des Wassers zu dem oder den organischen $C_1$-$C_6$-Alkoxysilanen (a1) in Gang gesestzt werden. Optional kann bei der Reaktion bzw. Hydrolyse ein Lösungsmittel zugegen sein.

Ebenfalls erfindungsgemäß ist ein Starten der Hydrolyse durch Vorlegen der Wassermenge (a2) und Hinzugeben der Aminoalkyl-$C_1$-$C_6$-alkoxysilane (a1).

Das Vermischen mit Wasser kann bei Raumtemperatur erfolgen. Für die anwendungstechnischen Eigenschaften des späteren kosmetischen Mittels kann es von weiterhin Vorteil sein, wenn das Gemisch aus organischen $C_1$-$C_6$-Alkoxysilanen (a1) und ggf. Lösungsmittel auf eine Temperatur von 30 bis 80 °C, bevorzugt von 40 bis 75°C, weiter bevorzugt von 45 bis 70 °C und ganz besonders bevorzugt von 50 bis 65 °C erwärmt wird, bevor das Wasser (a2) hinzugegeben wird.

Die Einstellung der bevorzugten und besonders bevorzugten Temperaturbereiche kann durch Temperieren des Reaktionsgefäßes oder Reaktors erfolgen. Beispielsweise kann das Reaktionsgefäß oder der Reaktor von außen mit einem Temperierbad umgeben werden, bei dem es sich beispielsweise um ein Wasserbad oder um Silikonölbad handeln kann.

Wird die Reaktion in einem Doppelwandreaktor durchgeführt, so kann auch eine temperierte Flüssigkeit durch den Raum geleitet werden, der durch die beiden Wandungen gebildet wird und der den Reaktionsraum umgibt.

Da die Hydrolyse-Reaktion exotherm ist, hat es sich als besonders vorteilhaft herausgestellt, das Reaktionsgemisch zur verbesserten Wärmeabfuhr zu rühren bzw. zu durchmischen. Besonders bevorzugt erfolgt die Zugabe des Wasser daher unter Rühren. Die nun durch Zugabe von Wasser initiierte Reaktion verläuft weiter exotherm, so dass das Reaktionsgemisch ohne weitere Energiezufuhr bei den oben angegebenen bevorzugten Temperaturbereichen bleibt oder sich auch noch weiter erwärmen kann. Es ist bevorzugt, wenn die durch die Exothermie der Reaktion bedingte zusätzliche Erwärmung innerhalb eines Bereichs von 5 bis 20 °C bleibt. Erwärmt sich das Reaktionsgemisch über diesen Bereich hinaus, ist es von Vorteil, das Gemisch zu kühlen.

Das Wasser kann kontinuierlich, in Teilmengen oder direkt als Gesamtmenge zugegeben werden. Um eine ausreichende Temperaturkontrolle zu gewährleisten, wird die Zugabemenge und -geschwindigkeit des Wassers bevorzugt angepasst. Je nach Menge der eingesetzten Silane kann die Zugabe und Reaktion über einen Zeitraum von 2 Minuten bis zu 72 Stunden erfolgen.

Die Zugabe des Wassers (a2) initiiert eine gezielte Hydrolyse der organischen $C_1$-$C_6$-Alkoxysilane (a1). Unter einer gezielten Hydrolyse ist im Sinne der vorliegenden Erfindung zu verstehen, dass ein Teil, aber nicht alle der in den organischen $C_1$-$C_6$-Alkoxysilanen vorhandenen $C_1$-$C_6$-Alkoxygruppen hydrolysiert werden.

Das Alkoxysilan-Wasser-Verhältnis hat dabei einen Einfluss auf die Vernetzung innerhalb des Siloxan-Netzwerkes. Der Vernetzungsgrad kann durch so genannte T-Strukturen beschrieben werden. Dabei steht $T_0$ für ein unreagiertes Monomer, z.B. 3-Aminopropyltriethoxysilan. Bei einer $T_1$-Bindung besteht zwischen zwei Alkoxysilanen eine Siloxan-Bindung, die beiden Siloxane sind zu keinem weiterem Alkoxysilan gebunden. Bei einer $T_2$-Bindung ist ein Alkoxysilan an genau zwei weitere gebunden. Eine $T_3$-Bindung beschreibt ein Siloxan welches zu drei weiteren Siloxanen gebunden ist.

Durch den Einsatz von unterschiedlichen Mol-Verhältnissen von Alkoxysilanen (a1) zu Wasser (a2) kann die Verteilung dieser verschiedenen T-Strukturen beeinflusst werden. Dabei führt ein hohes Verhältnis, also weniger Wasser, zu wenig vernetzten Strukturen, während hingegen ein niedriges Verhältnis, also eine größere Wassermenge, zu einer stärkeren Vernetzung führt.

Die quantitative Bestimmung der jeweils im Mittel enthaltenen T0-Strukturen, T1-Strukturen, T2-Strukturen und T3-Strukturen kann beispielswiese mittels quantitativer 29Si-NMR-Spektroskopie erfolgen.

- Verwendung von NMR-Probenröhrchen
- Gerät: Agilent, 600 MHz

Von den Zusammensetzungen wurden jeweils 29Si-NMR Spektren in Chloroform aufgenommen. Gemessen wurde am Tag der Herstellung sowie nach einem jeweils definierten Zeitraum.

- Standard: TMS (Tetramethylsilan)
- Relaxationsbeschleuniger: Chrom(III)acetylacetonat

Durch Einsatz des Relaxationsbeschleunigers wurden die Integrale der einzelnen Signale miteinander vergleichbar. Die Summe über alle Integrale wurde gleich 100 Mol-% gesetzt.

Für die quantitative Bestimmung wurde die Fläche jedes einzelnen Signals zur Gesamtsumme über alle Integrale in Relation gesetzt.

Die Messung der Spektren kann beispielsweise nach dem Verfahren durchgeführt werden, das in Journal of Organometallic Chemistry 625 (2001), 208-216 beschrieben ist.

Die zur Herstellung des Mittels (A) eingesetzte Wassermenge entspricht bevorzugt der Molmenge an Wasser, die sich nach der Gleichung (G-1) bestimmt

$$X = [(n_I(\text{Alkoxysilane (a1)}) \times n_{II}(\text{Alkoxygruppen})] / n(H_2O\ (a2)) \qquad (G\text{-}1)$$

wobei

| | |
|---|---|
| $n(H_2O)$ | die Molmenge des im Mittel (A) eingesetzten Wassers (a2) ist, |
| $n_I$ | die Molmenge der im Mittel (A) eingesetzten organischen $C_1$-$C_6$-Alkoxysilane (a1) ist, |
| $n_{II}$, | die Anzahl der $C_1$-$C_6$-Alkoxygruppenpro im Mittel (A) eingesetzten organischen $C_1$-$C_6$-Alkoxysilan (a1) ist, und |
| X | eine Zahl von 0,1 bis 100,0 darstellt. |

Mit anderen Worten gibt die Indexzahl X das molare Verhältnis aus der gesamten Molanzahl an hydrolysierbaren $C_1$-$C_6$-Alkoxygruppen(a1) im Mittel (A) an, die zur eingesetzten Molmenge an Wasser (a2) im Mittel (A) in Relation gesetzt wird.

nl gibt die gesamte Molmenge der im Mittel (A) eingesetzten organischen $C_1$-$C_6$-Alkoxysilane (a1) an. Wird nur ein $C_1$-$C_6$-Alkoxysilan einer bestimmten Struktur eingesetzt, so entspricht die Gesamtmolmenge $n_I$ der Molmenge des eingesetzten $C_1$-$C_6$-Alkoxysilans.

Wird zur Herstellung des Mittels (A) jedoch ein Gemisch aus $C_1$-$C_6$-Alkoxysilanen (a1) verwendet, so summiert sich die Gesamtmolmenge aus den jeweils einzelnen Molmengen eines jeden eingesetzten $C_1$-$C_6$-Alkoxysilans.

Weiterhin gibt $n_{II}$, die Anzahl der $C_1$-$C_6$-Alkoxygruppenpro im Mittel (A) eingesetzten organischen $C_1$-$C_6$-Alkoxysilan (a1) an. Wird nur ein $C_1$-$C_6$-Alkoxysilan einer bestimmten Struktur eingesetzt, so entspricht nII der Anzahl der in diesem Molekül vorhandenen $C_1$-$C_6$-Alkoxygruppen.

Wird zur Herstellung des Mittels (A) jedoch ein Gemisch aus $C_1$-$C_6$-Alkoxysilanen (a1) verwendet, so geht die Anzahl der $C_1$-$C_6$-Alkoxygruppen(a1) jedes einzelnen $C_1$-$C_6$-Alkoxysilan in die Gleichung ein.

Werden mehrere $C_1$-$C_6$-Alkoxysilane (a1) zur Herstellung des Mittel (A) eingesetzt, erweitert sich die o.g. Gleichung durch Ausbildung der jeweiligen Summmanden zur Gleichung (G-1'):

$$X = \frac{\sum[nI(Alkoxysilan\ (a1))\ x\ nII(Alkoxygruppen)]}{n\ (H2O)(a2)} \qquad \text{(G-1')}$$

Rechenbeispiel

Bei der Herstellung des Mittels (A) wurden 15,0 g 3-Aminopropyltriethoxsilan ($C_9H_{23}NO_3Si$ = 221,37 g/mol) und 15,0 g 3-Aminopropyltrimethoxysilan (C6H17NO3Si = 179,29 g/mol) miteinander vermischt. Anschließend wurden unter Rühren 6,06 g Wasser (18,015 g/mol) hinzugetropft.

15,0 g 3-Aminopropyltriethoxsilan (AMEO) = 0,068 mol
3-Aminopropyltriethoxsilan besitzt 3 hydrolysierbare Alkoxygruppen pro Molekül
15,0 g 3-Aminopropyltrimethoxysilan (AMMO) = 0,085 mol
3-Aminopropyltrimethoxysilan besitzt 3 hydrolysierbare Alkoxygruppen pro Molekül
3,0 g Wasser = 0,167 mol

Bei Einsatz von zwei verschiedenen $C_1$-$C_6$-Alkoxysilan berechnet sich der Wert X durch Anwendung der Formel (G-1') under Ausbildung entsprechender Summen, d.h.

$$X = \frac{[n(AMEO)x3] + (n(AMMO)x3]}{n\ (H2O)(a2)}$$

$$X = \frac{[(0{,}068)x3]+[(0{,}085)x3]}{0{,}167} = 2{,}75$$

Werden weitere bzw. andere Gemische an $C_1$-$C_6$-Alkoxy-Silanen (a1) eingesetzt, wird die Formel (G-1) bzw. (G-1') entsprechend adaptiert bzw. durch die entsprechenden Summanden erweitert.

Der bei Herstellung des Mittels (A) durch Zugabe der geeigneten Wassermenge (a2) eingestellte Vernetzungsgrad nimmt auf die anwendungstechnischen Eigenschaften des bei der späteren Anwendung auf dem Keratinmaterial erzeugten Coatings Einfluss. Ein besonders stabiler, und widerstandsfähiger Film konnte erzeugt werden, wenn erste Mittel (A) erhalten wird durch Reaktion mit einer Wassermenge, die der Molmenge an Wasser entspricht, die sich nach der Gleichung (G-1) bestimmt, wobei

X eine Zahl von 0,1 bis 100,0, bevorzugt von 1,0 bis 50,0 weiter bevorzugt von 1,2 bis 30,0, nochweiter bevorzugt von 1,3 bis 10 und ganz besonders bevorzugt von 1,8 bis 4,5 darstellt.

Im Rahmen einer weiteren besonders bevorzugten Ausführungsform ist ein erfindungsgemäßes Verfahren daher dadurch gekennzeichnet, dass die organischen $C_1$-$C_6$-Alkoxysilane (a1) im Mittel (A) mit einer Wassermenge (a2) zur Reaktion gebracht werden, die der Molmenge an Wasser entspricht, die sich nach der Gleichung (G-1) bestimmt,

$$X = [(n_I(\text{Alkoxysilane (a1)}) \times n_{II}(\text{Alkoxygruppen})] / n(H_2O\ (a2)) \qquad \text{(G-1)}$$

wobei

$n(H_2O)$ die Molmenge des im Mittel (A) eingesetzten Wassers (a2) ist,
$n_I$ die Molmenge der im Mittel (A) eingesetzten organischen $C_1$-$C_6$-Alkoxysilane (a1) ist,
$n_{II}$, die Anzahl der $C_1$-$C_6$-Alkoxygruppenpro im Mittel (A) eingesetzten organischen $C_1$-$C_6$-Alkoxysilan (a1) ist, und
X eine Zahl von 0,1 bis 100,0, bevorzugt von 1,0 bis 50,0 weiter bevorzugt von 1,2 bis 30,0, nochweiter bevorzugt von 1,3 bis 10 und ganz besonders bevorzugt von 1,8 bis 4,5 darstellt.

Einsatz von Katalysatoren (a3) zur gezielten Hydrolyse der organische $C_1$-$C_6$-Alkoxysilane (a1) im Mittel (A)

Im Rahmen der zu dieser Erfindung führenden Arbeiten hat sich herausgestellt, dass die im Mittel (A) eingesetzten Aminoalkyl-$C_1$-$C_6$-alkoxysilane (a1) so reaktiv sind, dass eine gezielte Hydrolyse- bzw. Vorkondensation mit

Wasser (a2) auch ohne Zuhilfenahme bzw. ohne Anwesenheit eines Katalysators mit ausreichend hoher Geschwindigeit abläuft.

Daher ist es bevorzugt, die Reaktion des bzw. der organischen $C_1$-$C_6$-Alkoxysilanen (a1) mit Wasser (a2) ohne Katalysator (a3) durchzuführen.

Prinzipiell wird der Einsatz eines Katalyators (a3) im Mittel (A) jedoch nicht ausgeschlossen. Unter einem Katalyator (a3) versteht der Fachmann einen Stoff, der die Reaktionsgeschwindigkeit durch die Senkung der Aktivierungsenergie einer chemischen Reaktion erhöht, ohne dabei selbst verbraucht zu werden. Die Zugabe des Katalysators (a3) kann vor oder nach Zugabe des Wassers (a2) erfolgen.

Für die Hydrolyse bzw. Vorkondensation der Gemische aus organischen $C_1$-$C_6$-Alkoxy-Siloxanen (a1) hat es sich als besonders vorteilhaft herausgestellt, einen Katalysator zu verwenden, der sich in Wasser lösen bzw. dispergieren lasst und dann zusammen mit dem Wasser als Lösung oder Dispersion zu dem Gemisch aus organischen $C_1$-$C_6$-Alkoxysilanen und Lösungsmittel hinzugegeben wird.

Ganz besonders bevorzugt wird der Katalysator (a3) ausgewählt aus der Gruppe der anorganischen oder organischen Säuren und der anorganischen oder organischen Basen.

Besonders gut geeignete Katalysaroten sind anorganische und organischen Säuren, die bevorzugt ausgewählt werden können aus der Gruppe aus Schwefelsäure, Salzsäure, Phosphorsäure, Maleinsäure, Zitronensäure, Weinsäure, Äpfelsäure, Milchsäure, Essigsäure, Methansulfonsäure, Benzoesäure, Malonsäure, Oxalsäure und 1-Hydroxyethan-1,1-diphosphonsäure. Explizit ganz besonders bevorzugt sind Schwefelsäure, Salzsäure und Maleinsäure.

Weiterhin besonders gut geeignete Katalysatoren sind anorganische und organischen Basen, die bevorzugt ausgewählt werden können aus der Gruppe aus Natriumhydroxid, Kaliumhydroxid, Magnesiumhydroxid und Calciumhydroxid. Ganz besonders bevorzugt sind Natriumhydroxid und Kaliumhydroxid.

Des Weiteren als Basen können beispielsweise Ammoniak, Alkanolamine und/oder basische Aminosäuren eingesetzt werden.

Alkanolamine können ausgewählt werden aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol.

Als Aminosäure im Sinne der Erfindung gilt eine organische Verbindung, die in ihrer Struktur mindestens eine protonierbare Aminogruppe und mindestens eine -COOH- oder eine $-SO_3H$-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können auch noch weitere anorganische Alkalisierungsmittel oder Basen eingesetzt werden. Erfindungsgemäß einsetzbare, anorganische Alkalisierungsmittel können beispielsweise ausgewählt werden aus der Gruppe, die gebildet wird aus Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass der Katalysator ausgewählt ist aus der Gruppe der anorganischen und der organischen Basen, bevorzugt aus der Gruppe aus Natriumhydroxid, Kaliumhydroxid, Magnesiumhydroxid und Calciumhydroxid.

In einer weiteren Ausführungsform ist eine erfindungsgemäßes Verfahren dadurch gekennzeichnet, dass

- das erste Mittel (A) erhalten wird durch Reaktion von einem oder mehreren organischen $C_1$-$C_6$-Alkoxysilanen (a1) mit Wasser (a2) und einem oder mehreren Katalysatoren (a3),
  wobei
- das oder die organischen $C_1$-$C_6$-Alkoxysilane (a1) ausgewählt sind aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)triethoxysilan, (3-Dimethylaminopropyl)tri-methoxysilan, (2-Dimethylaminoethyl)triethoxysilan und (2-Dimethyl-aminoethyl)-trimethoxysilan, und

- der oder die Katalysatoren ausgewählt (a3) ausgewählt sind aus der Gruppe aus anorganischen Säuren, organischen Säuren, anorganischen Basen und organischen Basen, bevorzugt ausgewählt aus der Gruppe aus Schwefelsäure, Salzsäure, Phosphorsäure, Maleinsäure, Zitronensäure, Weinsäure, Äpfelsäure, Milchsäure, Essigsäure, Methansulfonsäure, Benzoesäure, Malonsäure, Oxalsäure und 1-Hydroxyethan-1,1-diphosphonsäure, Natriumhydroxid, Kaliumhydroxid, Magnesiumhydroxid und Calciumhydroxid.

Erfindungsgemäß werden die Katalysatoren (a3) bevorzugt in den für Katalysatoren üblichen Mengenbereichen eingesetzt. Da die Katalysatoren (a3) die Hydrolyse- bzw. Kondensation beschleunigen, ohne dabei selbst verbraucht zu werden, können die Einsatzmengen entsprechend gering gewählt werden.

So können der oder die Katalysatoren (a3) in einem Mengenbereich von 0,0000001 bis 2,0 Gew.-%, bevorzugt von 0,0001 bis 1,5 Gew.-% und ganz besonders bevorzugt von 0,01 bis 1,0 Gew.-% im Mittel (A) eingesetzt werden. Hierbei bezieht sich die Angabe in Gew.-% auf die gesamte Einsatzmenge an Katalyatoren, die zu der Gesamtmenge des Mittels (A) in Relation gesetzt wird.

Abwesenheit von organischen $C_1$-$C_6$-Alkoxysilanen aus der Gruppe (b1) im Mittel (A)

Wesentlich für das erfindungsgemäße Verfahren ist die getrennte Herstellung der beiden Silan-Blends (A) und (B), wobei jedes der beiden Mittel $C_1$-$C_6$-Alkoxysilane einer bestimmten Gruppe enthält.

Im ersten Mittel (A) sind nur die Silane (a1) vom Typ der Aminoalkyl-$C_1$-$C_6$-alkoxysilane enthalten, die hydrolyisiert und miteinander vorkondensiert werden. Das zweite Mittel (B) hingegen enthält ausschließlich Silane (b1) vom Typ der Alkyl-$C_1$-$C_6$-alkoxysilane. Beide Silan-Typen (a1) und (b1) weisen unterschiedlich hohe Reaktivitäten auf, so dass durch die Trennung der beiden Silan-Typen voneinander eine verbesserte Steuerung der beiden Hydrolysereaktionen möglich wird.

Wurden nach der getrennten Hydrolyse dann die beiden Mittel (A) und (B) miteinander vermischt und die mit dieser Mischung erhaltene kosmetischen Zusammensetzung später auf dem Keratinmaterial angewendet, so konnte beobachtet werden, dass die hierbei erhaltenen Ergebnisse viel besser reproduzierbar waren. Wurde zum Beispiel die Mischung der Mittel (A) und (B) in einem Haarfärbeverfahren verwendet, so waren sowohl die Intensität der Färbung als auch ihre Echtheitseigenschaften viell besser reproduzierbar. Auf diese Weise gewährleistet die Anwendung der über das erfindungsgemäße Verfahren hergestellten Mischung aus (A) und (B) die Erzeugung von Haarfärbungen mit immer gleichem Farbresultat, unabhängig von den bei der Anwendung herrschenden klimatischen Bedingungen (wie z.B. Luftfeuchtigkeit und Temperatur), der Geübtheit bzw. Schnelligkeit des Anwenders und den Lagerzeiten der Mittel.

Um eine getrennte Hydrolyse der beiden Silan-Typen (a1) und (b1) voneinander durchführen zu können, ist es wesentlich, dass das Mittel (A) keine organischen $C_1$-$C_6$-Alkoxysilane aus der Gruppe (b1) enthält.

In anderen Worten ist das erfindungsgemäße Verfahren dadurch gekennzeichnet, dass das Mittel (A) kein organisches $C_1$-$C_6$-Alkoxysilan (b1 aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Vinyltrimethoxysilan, Vinyltriethoxysilan Tetramethoxysilan und Tetraethoxysilan enthält.

In anderen Worten ist das erfindungsgemäße Verfahren dadurch gekennzeichnet, dass die Gesamtmenge der im Mittel (A) enthaltenen organischen $C_1$-$C_6$-Alkoxysilan (b1) aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Vinyltrimethoxysilan, Vinyltriethoxysilan Tetramethoxysilan und Tetraethoxysilan - bezogen auf das Gesamtgewicht des Mittels (A) - unterhalb von 0,01 Gew.-%, bevorzugt unterhalb von 0,001 Gew.-% und ganz besonders bevorzugt bei 0 Gew.-% liegt.

weitere Inhaltsstoffe im Mittel (A)

Neben dem oder den organischen $C_1$-$C_6$-Alkoxysilanen (a1) und Wasser (a2) können im Mittel (A) optional auch noch ein oder mehrerweitere Inhaltsstoffe enthalten sein. So kann beipsielsweise die Hydrolyse der $C_1$-$C_6$-Alkoxysilanen (a1) in Anwesenheit eines oder mehrerer Lösungsmittel erfolgen.

Das Vermischen kann beispielsweise erfolgen, indem zunächst das von Wasser verschiedene Lösungsmittel in einem geeigneten Reaktor oder Reaktionsgefäß vorgelegt und dann das oder die organischen $C_1$-$C_6$-Alkoxysilane hinzugegeben werden. Die Zugabe kann durch Zutropfen oder Hinzugießen erfolgen. Weiterhin ist es ebenfalls möglich und erfindungsgemäß, wenn erst mindestens ein organisches $C_1$-$C_6$-Alkoxysilan im Reaktionsgefäß vorgelegt wird und danach das Lösungsmittel hinzugefügt bzw. zugetropft wird.

Auch eine sequentielle Vorgehensweise ist möglich, d.h. zunächst die Zugabe von Lösungsmittel und einem ersten organischen $C_1$-$C_6$-Alkoxysilan, danach wieder die Zugabe eines Lösungsmittels und dann wieder die Zugabe eines weiteren organischen $C_1$-$C_6$-Alkoxysilans.

Die Zugabe des Lösungsmittels erfolgt bevorzugt unter Rühren.

Es kann bevorzugt sein, ein Lösungsmittel auszuwählen,das bei Normaldruck (1013 hPa) einen Siedepunkt von 20 bis 90 °C, bevorzugt von 30 bis 85 °C und ganz besonders bevorzugt von 40 bis 80 °C besitzt.

Geeignete Lösungsmittel sind beispielsweise:

- Dichlormethan mit einem Siedepunkt von 40 °C ( 1013 mbar)
- Methanol mit einem Siedepunkt von 65 °C (1013 mbar)
- Tetrahydrofuran mit einem Siedepunkt von 65,8 °C (1013 mbar)
- Ethanol mit einem Siedepunkt von 78 °C (1013 mbar)
- Isopropanol mit einem Siedepunkt von 82 °C (1013 mbar)
- Acetonitril mit einem Siedepunkt von 82 °C (1013 mbar)

Weiterhin können ganz besonders bevorzugte Lösungsmittel aus der Gruppe der ein- oder mehrwertigen $C_1$-$C_{12}$-Alkohole ausgewählt sein. Ein- oder mehrwertige $C_1$-$C_{12}$-Alkohole sind Verbindungen mit einem bis zwölf Kohlenstoffatomen, die eine oder mehrer Hydroxy-Gruppen tragen. Weitere, von den Hydroxygruppen verschiedene funktionelle Gruppen sind erfindungsgemäß in den $C_1$-$C_{12}$-Alkoholen nicht vorhanden. Die $C_1$-$C_{12}$-Alkohole können aliphatisch oder aromatisch sein.

Als geeignete $C_1$-$C_{12}$-Alkohole können beispielsweise Methanol, Ethanol, n-Propanol, Isopropanol, n-Pentanol, n-Hexanol, Benzylalkohol, 2-Phenylethanol, 1,2-Propandiol, 1,3-Propandiol und Glycerin genannt werden. Ganz besonders gut geeignete $C_1$-$C_{12}$-Alkohole sind Methanol, Ethanol und Isopropanol.

Um die Hydrolyse der C1-C6-Alkoxysilane (a1) durch das Wasser (a2) jedoch so genau wie möglich steuern zu können, hat es sich als ganz besonders vorteilhaft herausgestellt, dem Mittel (A) entweder nur die weiteren Bestandteile zuzusetzen, welche mit den Silanen (a1) nicht in unverhersehbarer Weise reagieren können, ober aber diese weiteren optionalen Bestandteile nur in geringen bis sehr geringen Mengen dem Mittel (A) zuzusetzen. Aus diesem Grund ist es ganz besonders bevorzugt, wenn das Mittel (A) - bezogen auf sein Gesamtgewicht - zu mindestens 60 Gew.-%, bevorzugt von mindestens 70 Gew.-%, weiter bevorzugt von mindestens 80 Gew.-%, noch weiter bevorzugt von mindestens 90 Gew.-% und ganz besonders bevorzugt von mindestens 98 Gew.-% aus den Silanen der Gruppe (a1) und Wasser (a2) besteht.

Im Rahmen einer weiteren ganz besonders bevorzugten Ausführungsform ist ein erfindungsgemäßes Verfahren dadurch gekennzeichnet, dass die Gesamtmenge der im Mittel (A) eingesetzten Bestandteile (a1) und (a2) einen Gewichtsanteil von mindestens 60 Gew.-%, bevorzugt von mindestens 70 Gew.-%, weiter bevorzugt von mindestens 80 Gew.-%, noch weiter bevorzugt von mindestens 90 Gew.-% und ganz besonders bevorzugt von mindestens 98 Gew.-%, bezogen auf das Gesamtgewicht des Mittels (A), ausmacht.

Mittel (B)

Das das zweite Mittel (B) wird erhalten wird durch Reaktion von einem oder mehreren organischen $C_1$-$C_6$-Alkoxysilanen (b1) mit Wasser (b2).

Organische $C_1$-$C_6$-Alkoxy-Silane (b2) im Mittel (B)

Bei dem oder den organischen $C_1$-$C_6$-Alkoxy-Silanen (b1) handelt es sich um organische, nicht polymere Siliciumverbindungen, die aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Vinyltrimethoxysilan, Vinyltriethoxysilan Tetramethoxysilan und Tetraethoxysilan ausgewählt sind.

Die zur Lösung der erfindungsgemäßen Aufgabenstellung besonders gut geeigneten organischen $C_1$-$C_6$-Alkoxy-Silanen (b1) sind

- Methyltrimethoxysilan

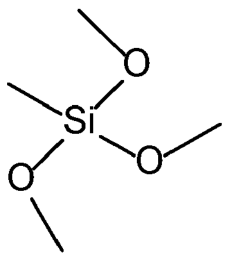

- Methyltriethoxysilan

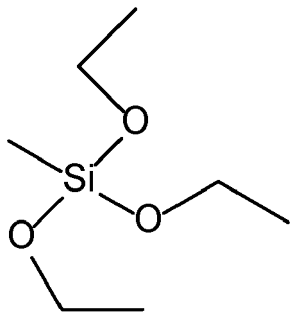

- Ethyltrimethoxysilan

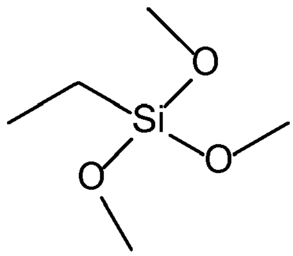

- Ethyltriethoxysilan

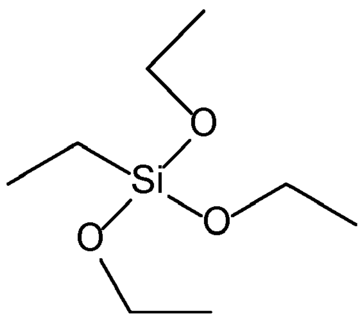

- n-Hexyltrimethoxysilan (auch bezeichnet als Hexyltrimethoxysilan)

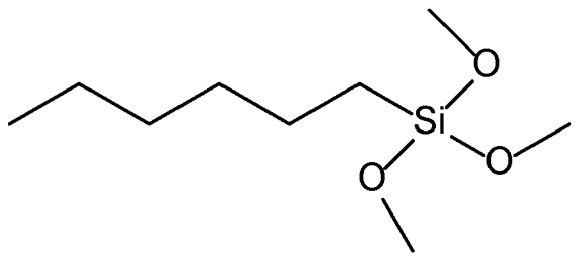

- n-Hexyltriethoxysilan (auch bezeichnet als Hexyltriethoxysilan)

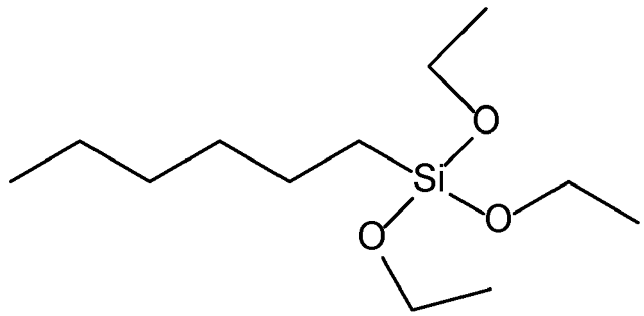

- n-Octyltrimethoxysilan (auch bezeichnet als Octyltrimethoxysilan)

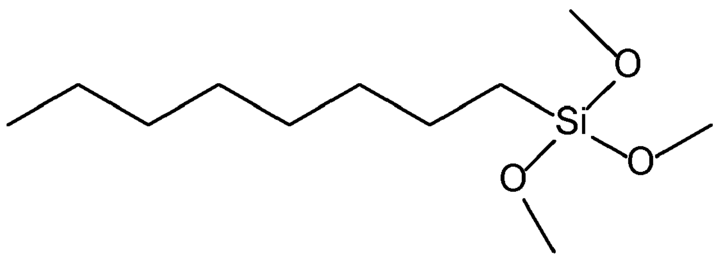

- n-Octyltriethoxysilan (auch bezeichnet als Octyltriethoxysilan)

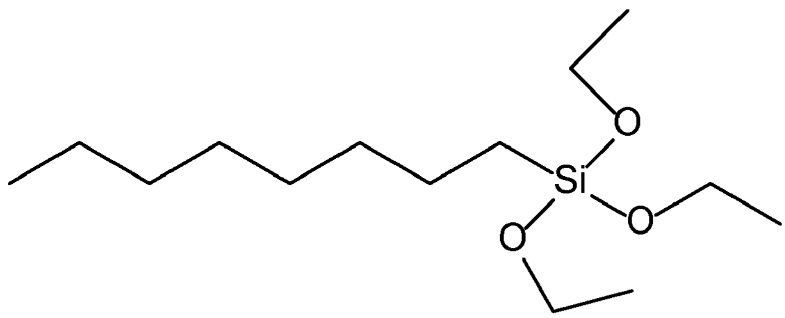

- n-Dodecyltrimethoxysilan (auch bezeichnet als Dodecyltrimethoxysilan) und/oder

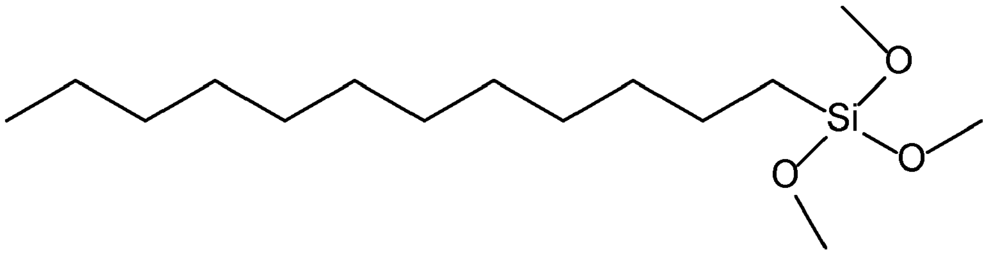

- n-Dodecyltriethoxysilan (auch bezeichnet als Dodecyltriethoxysilan).

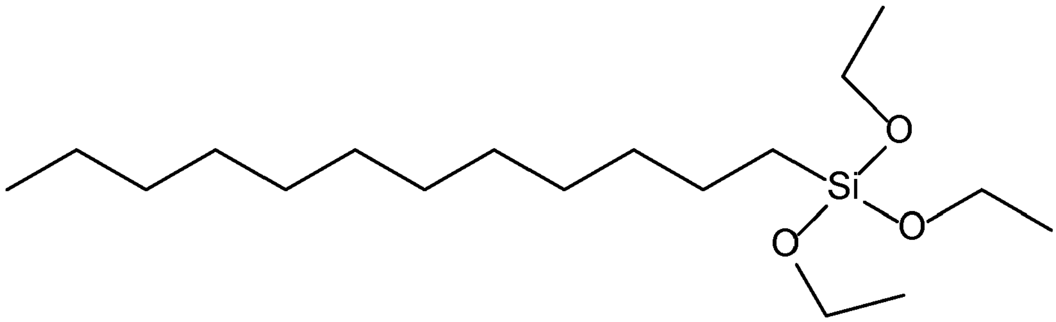

- Vinyltrimethoxysilan

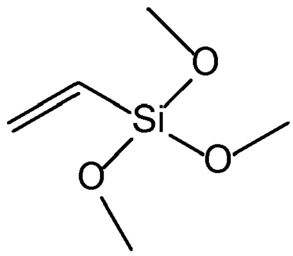

- Vinyltriethoxysilan

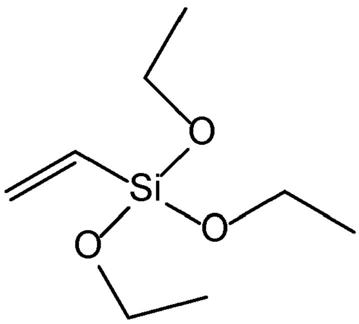

- Tetramethoxysilan

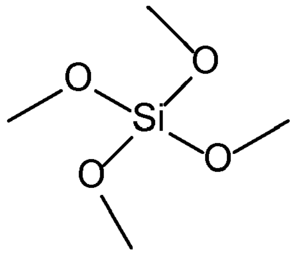

- Tetraethoxysilan

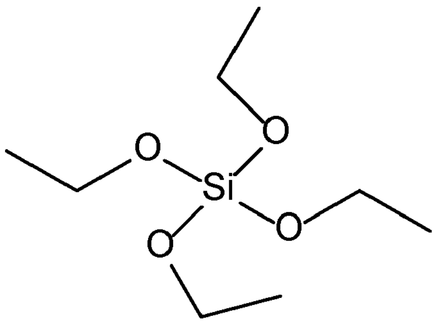

Auch bei der Herstellung des Mittels (B) kann entweder nur ein organisches $C_1$-$C_6$-Alkoxysilan aus der Gruppe (b1) oder auch die Gemische dier Gruppe eingesetzt werden.

Die Silane der Gruppe (b1) können kommerziell erworben werden. Zum Beispiel sind Methyltriethoxysilan und Methyltrimethoxysilan von Chemikalien-Lieferanten wie Acros, Sigma-Alrich, Fluka und VWR käuflich erhältlich.

Bei der Herstellung des ersten Mittels (B) werden eine oder mehrere organische $C_1$-$C_6$-Alkoxysilane (b1) mit Wasser (b2) zur Reaktion gebracht. Besonders gute Ergebnisse wurden erhalten, wenn als organische $C_1$-$C_6$-Alkoxysilane (b1) Methyltriethoxysilan und/oder Methyltri-methoxysilan eingesetzt wurde. Am allermeisten bevorzugt ist es daher, wenn das zweite Mittel (B) erhalten wird durch Reaktion von Methyltriethoxysilan und/oder Methyltrimethoxysilan (b1) mit Wasser (b2).

<u>gezielte Hydrolyse der organische $C_1$-$C_6$-Alkoxysilane (b1) im Mittel (B) durch Zugabe von Wasser</u>

Bei der Herstellung des Mittels (B) wird ein bzw. ein Gemisch aus mehreren organischen $C_1$-$C_6$-Alkoxysilanen (b1) mit Wasser (b2) versetzt, um eine gezielte Hydrolyse und hierdurch bedingt eine Vorkondensation in Gang zu setzen.

Wie bereits zuvor beschrieben erfolgt die Hydrolyse der Alkoxysilanen (b1) im Mittel (B) getrennt von den Silanen der Gruppe (a1) im Mittel (A). Da im Mittel (B) ausschließlich Alkyl-$C_1$-$C_6$-alkoxy-silane miteinander oligomerisieren, wird im Mittel (B) ein Oligomer gebildet, welches ausschließlich durch gezielte Hydrolyse und Kondensation der Alkyl-$C_1$-$C_6$-alkoxysilane ausgebildet wird. Damit umfassen alle Monomerbestandteile des im Mittel (B) vorhandenen Oligomers bzw. Reaktions-produktes keine Aminoalkylgruppen, sondern nur Alkyl-Gruppen.

Die Reaktion mit dem Wasser (b2) kann beispielsweise durch Zutropfen oder Zugießen des Wassers zu dem oder den organischen $C_1$-$C_6$-Alkoxysilanen (b1) in Gang gesestzt werden. Optional kann bei der Reaktion bzw. Hydrolyse ein Lösungsmittel zugegen sein.

Ebenfalls erfindungsgemäß ist ein Starten der Hydrolyse durch Vorlegen der Wassermenge (b2) und Hinzugeben der Alkyl-$C_1$-$C_6$-alkoxy-silane (b1).

Das Vermischen mit Wasser kann bei Raumtemperatur erfolgen. Für die anwendungstechnischen Eigenschaften des späteren kosmetischen Mittels kann es von weiterhin Vorteil sein, wenn das Gemisch aus organischen $C_1$-$C_6$-Alkoxysilanen (b1) und ggf. Lösungsmittel auf eine Temperatur von 30 bis 80 °C, bevorzugt von 40 bis 75°C, weiter bevorzugt von 45 bis 70 °C und ganz besonders bevorzugt von 50 bis 65 °C erwärmt wird, bevor das Wasser (b2) hinzugegeben wird.

Die Einstellung der bevorzugten und besonders bevorzugten Temperaturbereiche kann durch Temperieren des Reaktionsgefäßes oder Reaktors erfolgen. Beispielsweise kann das Reaktionsgefäß oder der Reaktor von außen mit einem Temperierbad umgeben werden, bei dem es sich beispielsweise um ein Wasserbad oder um Silikonölbad handeln kann.

Wird die Reaktion in einem Doppelwandreaktor durchgeführt, so kann auch eine temperierte Flüssigkeit durch den Raum geleitet werden, der durch die beiden Wandungen gebildet wird und der den Reaktionsraum umgibt.

Da die Hydrolyse-Reaktion exotherm ist, hat es sich als besonders vorteilhaft herausgestellt, das Reaktionsgemisch zur verbesserten Wärmeabfuhr zu rühren bzw. zu durchmischen. Besonders bevorzugt erfolgt die Zugabe des Wasser daher unter Rühren. Die nun durch Zugabe von Wasser und gegebenenfalls einem Katalysator initiierte Reaktion verläuft weiter exotherm, so dass das Reaktionsgemisch ohne weitere Energiezufuhr bei den oben angegebenen bevorzugten Temperaturbereichen bleibt oder sich auch noch weiter erwärmen kann. Es ist bevorzugt, wenn die durch die Exothermie der Reaktion bedingte zusätzliche Erwärmung innerhalb eines Bereichs von 5 bis 20 °C bleibt. Erwärmt sich das Reaktionsgemisch über diesen Bereich hinaus, ist es von Vorteil, das Gemisch zu kühlen.

Das Wasser kann kontinuierlich, in Teilmengen oder direkt als Gesamtmenge zugegeben werden. Um eine ausreichende Temperaturkontrolle zu gewährleisten, wird die Zugabemenge und - geschwindigkeit des Wassers bevorzugt angepasst. Je nach Menge der eingesetzten Silane kann die Zugabe und Reaktion über einen Zeitraum von 2 Minuten bis zu 72 Stunden erfolgen.

Die Zugabe des Wassers (b2) initiiert eine gezielte Hydrolyse der organischen $C_1$-$C_6$-Alkoxysilane (b1). Unter einer gezielten Hydrolyse ist im Sinne der vorliegenden Erfindung zu verstehen, dass ein Teil, aber nicht alle der in den organischen $C_1$-$C_6$-Alkoxysilanen vorhandenen $C_1$-$C_6$-Alkoxygruppen hydrolysiert werden.

Auch bei der Herstellung des Mittels (B) hat das Alkoxysilan-Wasser-Verhältnis einen Einfluss auf die Vernetzung innerhalb des Siloxan-Netzwerkes. Der Vernetzungsgrad kann durch so genannte T-Strukturen beschrieben werden. Dabei steht $T_0$ für ein unreagiertes Monomer, z.B. Methyltriethoxysilan oder Methyltrimethoxysilan. Bei einer $T_1$ -Bindung besteht zwischen zwei Alkoxysilanen eine Siloxan-Bindung, die beiden Siloxane sind zu keinem weiterem Alkoxysilan gebunden. Bei einer $T_2$-Bindung ist ein Alkoxysilan an genau zwei weitere gebunden. Eine $T_3$-Bindung beschreibt ein Siloxan welches zu drei weiteren Siloxanen gebunden ist.

Durch den Einsatz von unterschiedlichen Mol-Verhältnissen von Alkoxysilanen (b1) zu Wasser (b2) kann die Verteilung dieser verschiedenen T-Strukturen beeinflusst werden. Dabei führt ein hohes Verhältnis, also weniger Wasser, zu wenig vernetzten Strukturen, während hingegen ein niedriges Verhältnis, also eine größere Wassermenge, zu einer stärkeren Vernetzung führt.

Die quantitative Bestimmung der jeweils im Mittel (B) enthaltenen T0-Strukturen, T1-Strukturen, T2-Strukturen und T3-Strukturen kann beispielswiese mittels quantitativer 29Si-NMR-Spektroskopie erfolgen.

- Verwendung von NMR-Probenröhrchen
- Gerät: Agilent, 600 MHz

Von den Zusammensetzungen wurden jeweils 29Si-NMR Spektren in Chloroform aufgenommen. Gemessen wurde am Tag der Herstellung sowie nach einem jeweils definierten Zeitraum.

- Standard: TMS (Tetramethylsilan)
- Relaxationsbeschleuniger: Chrom(III)acetylacetonat

Durch Einsatz des Relaxationsbeschleunigers wurden die Integrale der einzelnen Signale miteinander vergleichbar. Die Summe über alle Integrale wurde gleich 100 Mol-% gesetzt.

Für die quantitative Bestimmung wurde die Fläche jedes einzelnen Signals zur Gesamtsumme über alle Integrale in Relation gesetzt.

Die Messung der Spektren kann beispielsweise nach dem Verfahren durchgeführt werden, das in Journal of Organometallic Chemistry 625 (2001), 208-216 beschrieben ist.

Die zur Herstellung des Mittels (B) eingesetzte Wassermenge entspricht bevorzugt der Molmenge an Wasser, die sich nach der Gleichung (G-2) bestimmt

$$Y = [(m_I(\text{Alkoxysilane (b1)}) \times m_{II}(\text{Alkoxygruppen})] / m(H_2O\text{ (b2)}) \quad \text{(G-2)}$$

wobei

| | |
|---|---|
| $m(H_2O)$ | die Molmenge des im Mittel (B) eingesetzten Wassers (b2) ist, |
| $M_I$ | die Molmenge der im Mittel (B) eingesetzten organischen $C_1$-$C_6$-Alkoxysilane (b1) ist, |
| $M_{II}$ | die Anzahl der $C_1$-$C_6$-Alkoxygruppenpro im Mittel (B) eingesetzten organischen $C_1$-$C_6$-Alkoxysilan (b1) ist, und |
| Y | eine Zahl von darstellt. |

Mit anderen Worten gibt die Indexzahl Y das molare Verhältnis aus der gesamten Molanzahl an hydrolysierbaren $C_1$-$C_6$-Alkoxygruppen(b1) im Mittel (B) an, die zur eingesetzten Molmenge an Wasser (b2) im Mittel (B) in Relation

gesetzt wird.

$m_I$ gibt die gesamte Molmenge der im Mittel (B) eingesetzten organischen $C_1$-$C_6$-Alkoxysilane (b1) an. Wird nur ein $C_1$-$C_6$-Alkoxysilan (b1) einer bestimmten Struktur eingesetzt, so entspricht die Gesamtmolmenge $n_I$ der Molmenge des eingesetzten $C_1$-$C_6$-Alkoxysilans.

Wird zur Herstellung des Mittels (B) jedoch ein Gemisch aus $C_1$-$C_6$-Alkoxysilanen (b1) verwendet, so summiert sich die Gesamtmolmenge aus den jeweils einzelnen Molmengen eines jeden eingesetzten $C_1$-$C_6$-Alkoxysilans.

Weiterhin gibt $m_{II}$ die Anzahl der $C_1$-$C_6$-Alkoxygruppenpro im Mittel (B) eingesetzten organischen $C_1$-$C_6$-Alkoxysilan (b1) an. Wird nur ein $C_1$-$C_6$-Alkoxysilan einer bestimmten Struktur eingesetzt, so entspricht mII der Anzahl der in diesem Molekül vorhandenen $C_1$-$C_6$-Alkoxygruppen.

Wird zur Herstellung des Mittels (B) jedoch ein Gemisch aus $C_1$-$C_6$-Alkoxysilanen (b1) verwendet, so geht die Anzahl der $C_1$-$C_6$-Alkoxygruppen(b1) jedes einzelnen $C_1$-$C_6$-Alkoxysilan in die Gleichung ein.

Werden mehrere $C_1$-$C_6$-Alkoxysilane (b1) zur Herstellung des Mittel (B) eingesetzt, erweitert sich die o.g. Gleichung durch Ausbildung der jeweiligen Summmanden zur Gleichung (G-2'):

$$Y = \frac{\sum[mI(Alkoxysilan\ (b1))\ x\ mII(Alkoxygruppen)]}{m\ (H2O)(b2)} \qquad \text{(G-2')}$$

<u>Rechenbeispiel</u>

Bei der Herstellung des Mittels (B) wurden 15,0 g Methyltriethoxysilan (C7H18O3Si = 178,3 g/mol) und 15,0 g Methyltrimethoxysilan (C4H12O3Si = 136,22 g/mol) miteinander vermischt. Anschließend wurden unter Rühren 5,0 g Wasser (18,015 g/mol) hinzugetropft.

15,0 g Methyltriethoxysilan (MTES) = 0,0841 mol
Methyltriethoxysilan besitzt 3 hydrolysierbare Alkoxygruppen pro Molekül
15,0 g Methyltrimethoxysilan (MTMS) = 0,110 mol
Methyltrimethoxysilan besitzt 3 hydrolysierbare Alkoxygruppen pro Molekül.
5,0 g Wasser = 0,277 mol

Bei Einsatz von zwei verschiedenen $C_1$-$C_6$-Alkoxysilanen (b1) berechnet sich der Wert Y durch Anwendung der Formel (G-2') under Ausbildung entsprechender Summen, d.h.

$$Y = \frac{[m(MTES)x3] + (m(MTMS)x3]}{m\ (H2O)(b2)}$$

$$Y = \frac{[(0{,}0841)x3]+[(0{,}110)x3]}{0{,}277} = 2{,}10$$

Werden weitere bzw. andere Gemische an $C_1$-$C_6$-Alkoxy-Silanen (b1) eingesetzt, wird die Formel (G-2) bzw. (G-2') entsprechend adaptiert bzw. durch die entsprechenden Summanden erweitert.

Der bei Herstellung des Mittels (B) durch Zugabe der geeigneten Wassermenge (b2) eingestellte Vernetzungsgrad nimmt auf die anwendungstechnischen Eigenschaften des bei der späteren Anwendung auf dem Keratinmaterial erzeugten Coatings Einfluss. Ein besonders stabiler, und widerstandsfähiger Film konnte erzeugt werden, wenn zweite Mittel (B) erhalten wird durch Reaktion mit einer Wassermenge, die der Molmenge an Wasser entspricht, die sich nach der Gleichung (G-2) bestimmt, wobei

Y eine Zahl von 0,1 bis 100,0, bevorzugt von 1,0 bis 50,0 weiter bevorzugt von 1,2 bis 30,0, nochweiter bevorzugt von 1,3 bis 10 und ganz besonders bevorzugt von 1,8 bis 4,5 darstellt.

Im Rahmen einer weiteren besonders bevorzugten Ausführungsform ist ein erfindungsgemäßes Verfahren daher dadurch gekennzeichnet, dass die organischen $C_1$-$C_6$-Alkoxysilane (b1) im Mittel (B) mit einer Wassermenge (b2) zur Reaktion gebracht werden, die der Molmenge an Wasser entspricht, die sich nach der Gleichung (G-2) bestimmt,

$$Y = [(m_I(\text{Alkoxysilane (b1)}) \times m_{II}(\text{Alkoxygruppen})] / m(H_2O\ (b2)) \quad (G\text{-}2)$$

wobei

| | |
|---|---|
| $m(H_2O)$ | die Molmenge des im Mittel (B) eingesetzten Wassers (b2) ist, |
| $m_I$ | die Molmenge der im Mittel (B) eingesetzten organischen $C_1$-$C_6$-Alkoxysilane (b1) ist, |
| $M_{II}$ | die Anzahl der $C_1$-$C_6$-Alkoxygruppenpro im Mittel (B) eingesetzten organischen $C_1$-$C_6$-Alkoxysilan (b1) ist, und |
| Y | eine Zahl von 0,1 bis 100,0, bevorzugt von 1,0 bis 50,0 weiter bevorzugt von 1,2 bis 30,0, nochweiter bevorzugt von 1,3 bis 10 und ganz besonders bevorzugt von 1,8 bis 4,5 darstellt. |

Einsatz von Katalysatoren (b3) zur gezielten Hydrolyse der organische $C_1$-$C_6$-Alkoxysilane (b1) im Mittel (B)

Wie die zu dieser Erfindung führenden Arbeiten gezeigt haben, besitzen die Alkyl-$C_1$-$C_6$-Alkoxy-silane (b1) im Mittel (B) eine etwas geringere Reaktivität als die Aminoalkyl-$C_1$-$C_6$-Alkoxsilane (a1) im Mittel (A). Während die Hydrolyse der reaktiveren Silane (a1) im Mittel (A) auch ohne Katalysator (a3) sehr schnell startet und in einer ausreichend hohen Geschwindigkeit abläuft, hat sich der Einsatz eines Katalysators (b3) im Mittel (B) zum ausreichend schnellen Start der Hydrolyse-Reaktion als bevorzugt herausgestellt.

Im Rahmen einer weiteren besonders bevorzugten Ausführungsform ist ein erfindungsgemäßes Verfahren daher dadurch gekennzeichnet, dass das zweite Mittel (B) erhalten wird durch Reaktion von einem oder mehreren organischen $C_1$-$C_6$-Alkoxysilanen (b1) mit Wasser (b2) und mindestens einem Katalysator (b3).

Auch der Katalyator (b3) wird als ein Stoff oder eine Substanz verstanden, welche die Reaktionsgeschwindigkeit durch die Senkung der Aktivierungsenergie einer chemischen Reaktion erhöht, ohne dabei selbst verbraucht zu werden. Die Zugabe des Katalysators (b3) kann vor oder nach Zugabe des Wassers (b2) erfolgen.

Für die Hydrolyse bzw. Vorkondensation der Gemische aus organischen $C_1$-$C_6$-Alkoxy-Siloxanen (b1) hat es sich als besonders vorteilhaft herausgestellt, einen Katalysator zu verwenden, der sich in Wasser lösen bzw. dispergieren lasst und dann zusammen mit dem Wasser als Lösung oder Dispersion zu dem Gemisch aus organischen $C_1$-$C_6$-Alkoxysilanen und Lösungsmittel hinzugegeben wird.

Ganz besonders bevorzugt wird der Katalysator (b3) ausgewählt aus der Gruppe der anorganischen oder organischen Säuren und der anorganischen oder organischen Basen.

Besonders gut geeignete Katalysaroten sind anorganische und organischen Säuren, die bevorzugt ausgewählt werden können aus der Gruppe aus Schwefelsäure, Salzsäure, Phosphorsäure, Maleinsäure, Zitronensäure, Weinsäure, Äpfelsäure, Milchsäure, Essigsäure, Methansulfonsäure, Benzoesäure, Malonsäure, Oxalsäure und 1-Hydroxyethan-1,1-diphosphonsäure. Explizit ganz besonders bevorzugt sind Schwefelsäure, Salzsäure und Maleinsäure.

Weiterhin besonders gut geeignete Katalysatoren sind anorganische und organischen Basen, die bevorzugt ausgewählt werden können aus der Gruppe aus Natriumhydroxid, Kaliumhydroxid, Magnesiumhydroxid und Calciumhydroxid. Ganz besonders bevorzugt sind Natriumhydroxid und Kaliumhydroxid.

Des Weiteren als Basen können beispielsweise Ammoniak, Alkanolamine und/oder basische Aminosäuren eingesetzt werden.

Alkanolamine können ausgewählt werden aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol.

Als Aminosäure im Sinne der Erfindung gilt eine organische Verbindung, die in ihrer Struktur mindestens eine protonierbare Aminogruppe und mindestens eine -COOH- oder eine -$SO_3H$-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel

um eine basische Aminosäure aus der Gruppe Arginin, Lysin, Ornithin und/oder Histidin handelt.

Darüber hinaus können auch noch weitere anorganische Alkalisierungsmittel oder Basen eingesetzt werden. Erfindungsgemäß einsetzbare, anorganische Alkalisierungsmittel können beispielsweise ausgewählt werden aus der Gruppe, die gebildet wird aus Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass der Katalysator ausgewählt ist aus der Gruppe der anorganischen und der organischen Basen, bevorzugt aus der Gruppe aus Natriumhydroxid, Kaliumhydroxid, Magnesiumhydroxid und Calciumhydroxid.

In einer weiteren Ausführungsform ist eine erfindungsgemäßes Verfahren dadurch gekennzeichnet, dass das zweite Mittel (B) erhalten wird durch Reaktion von einem oder mehreren organischen $C_1$-$C_6$-Alkoxysilanen (b1) mit Wasser (b2) und mindestens einem Katalysator (b3), wobei der Katalysator (b3) ausgewählt ist aus der Gruppe anorganischen und organischen Säuren, bevorzugt aus der Gruppe aus Schwefelsäure, Salzsäure, Phosphor-säure, Maleinsäure, Zitronensäure, Weinsäure, Äpfelsäure, Milchsäure, Essigsäure, Methan-sulfonsäure, Benzoesäure, Malonsäure, Oxalsäure und 1-Hydroxyethan-1,1-diphosphonsäure.

Erfindungsgemäß werden die Katalysatoren (b3) bevorzugt in den für Katalysatoren üblichen Mengenbereichen eingesetzt. Da die Katalysatoren (b3) die Hydrolyse- bzw. Kondensation beschleunigen, ohne dabei selbst verbraucht zu werden, können die Einsatzmengen entsprechend gering gewählt werden.

So können der oder die Katalysatoren (b3) in einem Mengenbereich von 0,0000001 bis 2,0 Gew.-%, bevorzugt von 0,0001 bis 1,5 Gew.-% und ganz besonders bevorzugt von 0,01 bis 1,0 Gew.-% im Mittel (B) eingesetzt werden. Hierbei bezieht sich die Angabe in Gew.-% auf die gesamte Einsatzmenge an Katalyatoren, die zu der Gesamtmenge des Mittels (B) in Relation gesetzt wird.

<u>Abwesenheit von organischen $C_1$-$C_6$-Alkoxysilane aus der Gruppe (b1) im Mittel (A)</u>

Wesentlich für das erfindungsgemäße Verfahren ist die getrennte Herstellung der beiden Silan-Blends (A) und (B), wobei jedes der beiden Mittel $C_1$-$C_6$-Alkoxysilane einer bestimmten Gruppe enthält.

So wie im ersten Mittel (A) nur die Silane (a1) vom Typ der Aminoalkyl-$C_1$-$C_6$-alkoxysilane enthalten, die hydrolysiert und miteinander vorkondensiert werden, so umfasst das zweite Mittel (B) ausschließlich Silane (b1) vom Typ der Alkyl-$C_1$-$C_6$-alkoxysilane.

Kennzeichnend für das erfindungsgemäße Verfahren ist demnach, dass das Mittel (B) keine organischen $C_1$-$C_6$-Alkoxysilane aus der Gruppe (a1) enthält.

In anderen Worten ist das erfindungsgemäße Verfahren dadurch gekennzeichnet, dass das Mittel (B) kein organisches $C_1$-$C_6$-Alkoxysilan (a1) aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)triethoxysilan, (3-Dimethylaminopropyl)tri-methoxysilan, (2-Dimethylaminoethyl)triethoxysilan und (2-Dimethyl-aminoethyl)-trimethoxysilan enthält.

In noch anderen Worten ist das erfindungsgemäße Verfahren dadurch gekennzeichnet, dass die Gesamtmenge der im Mittel (B) enthaltenen organischen $C_1$-$C_6$-Alkoxysilane (a1) aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)triethoxysilan, (3-Dimethylamino-propyl)trimethoxysilan, (2-Dimethylaminoethyl)triethoxysilan und (2-Dimethyl-aminoethyl)-trimethoxysilan - bezogen auf das Gesamtgewicht des Mittels (B) - unterhalb von 0,01 Gew.-%, bevorzugt unterhalb von 0,001 Gew.-% und ganz besonders bevorzugt bei 0 Gew.-% liegt

<u>weitere Inhaltsstoffe im Mittel (B)</u>

Neben dem oder den organischen $C_1$-$C_6$-Alkoxysilanen (b1) und Wasser (b2) können wie im Mittel (A) auch im Mittel (B) optional auch noch ein oder mehrer weitere Inhaltsstoffe enthalten sein. So kann beipsielesweise die Hydrolyse der $C_1$-$C_6$-Alkoxysilanen (b1) in Anwesenheit eines oder mehrerer Lösungsmittel erfolgen.

Das Vermischen kann beispielsweise erfolgen, indem zunächst das von Wasser verschiedene Lösungsmittel in einem geeigneten Reaktor oder Reaktionsgefäß vorgelegt und dann das oder die organischen $C_1$-$C_6$-Alkoxysilane hinzugegeben werden. Die Zugabe kann durch Zutropfen oder Hinzugießen erfolgen. Weiterhin ist es ebenfalls möglich und erfindungsgemäß, wenn erst mindestens ein organisches $C_1$-$C_6$-Alkoxysilan im Reaktionsgefäß vorgelegt wird und danach das Lösungsmittel hinzugefügt bzw. zugetropft wird.

Auch eine sequentielle Vorgehensweise ist möglich, d.h. zunächst die Zugabe von Lösungsmittel und einem ersten organischen $C_1$-$C_6$-Alkoxysilan, danach wieder die Zugabe eines Lösungsmittels und dann wieder die Zugabe eines weiteren organischen $C_1$-$C_6$-Alkoxysilans.

Die Zugabe des Lösungsmittels erfolgt bevorzugt unter Rühren.

Es kann bevorzugt sein, ein Lösungsmittel auszuwählen,das bei Normaldruck (1013 hPa) einen Siedepunkt von 20 bis 90 °C, bevorzugt von 30 bis 85 °C und ganz besonders bevorzugt von 40 bis 80 °C besitzt.

Geeignete Lösungsmittel sind beispielsweise:

- Dichlormethan mit einem Siedepunkt von 40 °C ( 1013 mbar)
- Methanol mit einem Siedepunkt von 65 °C (1013 mbar)
- Tetrahydrofuran mit einem Siedepunkt von 65,8 °C (1013 mbar)
- Ethanol mit einem Siedepunkt von 78 °C (1013 mbar)
- Isopropanol mit einem Siedepunkt von 82 °C (1013 mbar)
- Acetonitril mit einem Siedepunkt von 82 °C (1013 mbar)

Weiterhin können ganz besonders bevorzugte Lösungsmittel aus der Gruppe der ein- oder mehrwertigen $C_1$-$C_{12}$-Alkohole ausgewählt sein. Ein- oder mehrwertige $C_1$-$C_{12}$-Alkohole sind Verbindungen mit einem bis zwölf Kohlenstoffatomen, die eine oder mehrer Hydroxy-Gruppen tragen. Weitere, von den Hydroxygruppen verschiedene funktionelle Gruppen sind erfindungsgemäß in den $C_1$-$C_{12}$-Alkoholen nicht vorhanden. Die $C_1$-$C_{12}$-Alkohole können aliphatisch oder aromatisch sein.

Als geeignete $C_1$-$C_{12}$-Alkohole können beispielsweise Methanol, Ethanol, n-Propanol, Isopropanol, n-Pentanol, n-Hexanol, Benzylalkohol, 2-Phenylethanol, 1,2-Propandiol, 1,3-Propandiol und Glycerin genannt werden. Ganz besonders gut geeignete $C_1$-$C_{12}$-Alkohole sind Methanol, Ethanol und Isopropanol.

Um die Hydrolyse der $C_1$-$C_6$-Alkoxysilane (b1) durch das Wasser (b2) jedoch so genau wie möglich steuern zu können, hat es sich als ganz besonders vorteilhaft herausgestellt, dem Mittel (B) entweder nur die weiteren Bestandteile zuzusetzen, welche mit den Silanen (b1) nicht in unverhersehbarer Weise reagieren können, ober aber weitere Bestandteile nur in geringen bis sehr geringen Mengen dem Mittel (B) zuzusetzen. Aus diesem Grund ist es ganz besonders bevorzugt, wenn das Mittel (B) - bezogen auf sein Gesamtgewicht - zu mindestens 60 Gew.-%, bevorzugt von mindestens 70 Gew.-%, weiter bevorzugt von mindestens 80 Gew.-%, noch weiter bevorzugt von mindestens 90 Gew.-% und ganz besonders bevorzugt von mindestens 98 Gew.-% aus den Silanen der Gruppe (b1) und Wasser besteht.

Im Rahmen einer weiteren ganz besonders bevorzugten Ausführungsform ist ein erfindungsgemäßes Verfahren dadurch gekennzeichnet, dass die Gesamtmenge der im Mittel (B) eingesetzten Bestandteile (b1) und (b2) einen Gewichtsanteil von mindestens 60 Gew.-%, bevorzugt von mindestens 70 Gew.-%, weiter bevorzugt von mindestens 80 Gew.-%, noch weiter bevorzugt von mindestens 90 Gew.-% und ganz besonders bevorzugt von mindestens 98 Gew.-%, bezogen auf das Gesamtgewicht des Mittels (B), ausmacht.

Reaktion der organischen $C_1$-$C_6$-Alkoxy-Silane mit Wasser in den Mitteln (A) und (B)

In den Mitteln (A) und (B) kann die Reaktion der organischen $C_1$-$C_6$-Alkoxy-Silane mit Wasser auf verschiedenen Wegen stattfinden. Die Reaktion startet, sobald die $C_1$-$C_6$-Alkoxy-Silane mit Wasser durch Vermischen in Kontakt kommen. Sobald $C_1$-$C_6$-Alkoxy-Silane und Wasser in Kontakt kommen, findet eine exotherme Hydrolyse-Reaktion gemäß folgendem Schema statt

Mittel (A): Reaktionsschema am Beispiel von 3-Aminopropyltriethoxysilan

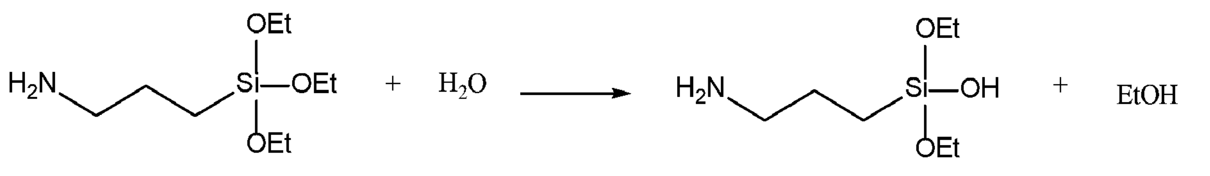

In Abhängigkeit von der Anzahl der hydrolysierbaren $C_1$-$C_6$-Alkoxygruppenpro Silan-Molekül kann die Hydrolysereaktion auch mehrfach pro eingesetztem $C_1$-$C_6$-Alkkoxy-Silan stattfinden:

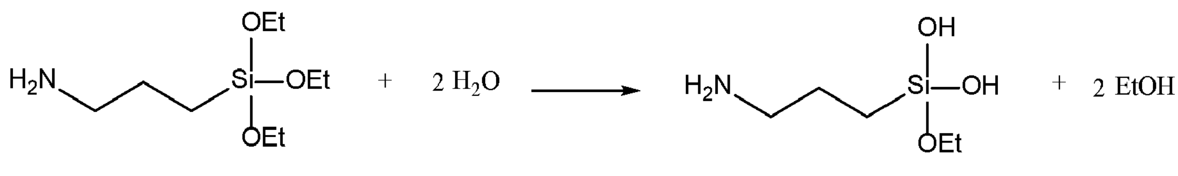

bzw.

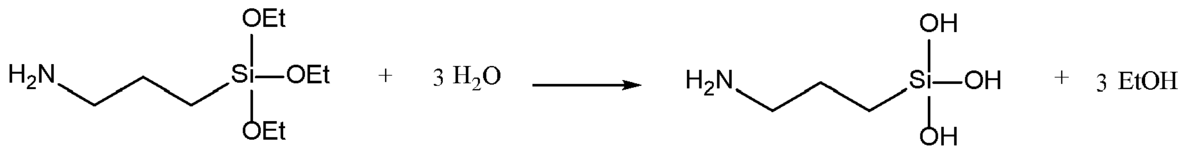

Mittel (B): Hydrolyse am Beispiel von Methyltrimethoxysilan:

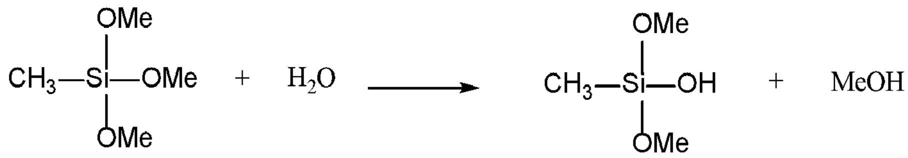

In Abhängigkeit von der eingesetzten Menge an Wasser kann die Hydrolyse-Reaktion auch mehrfach pro eingesetztem $C_1$-$C_6$-Alkoxy-Silan stattfinden:

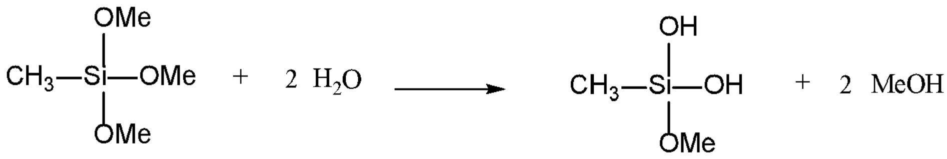

bzw.

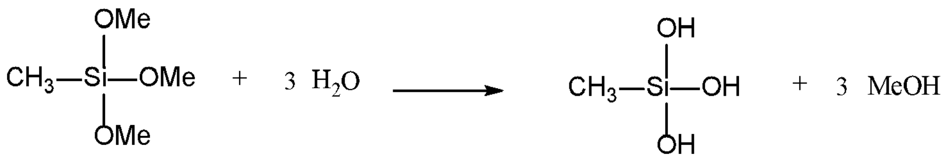

Im Anschluss an die Hydrolyse bzw. quasi zeitgleich mit der Hydrolyse erfolgt eine Kondensation der partiell (oder in Teilen auch vollständig) hydrolysierten $C_1$-$C_6$-Alkoxy-Silane.Die Vorkondensation kann beispielsweise gemäß dem folgenden Schema ablaufen:

Mittel (A): Kondensation am Beispiel von 3-Aminopropyltriethoxysilan

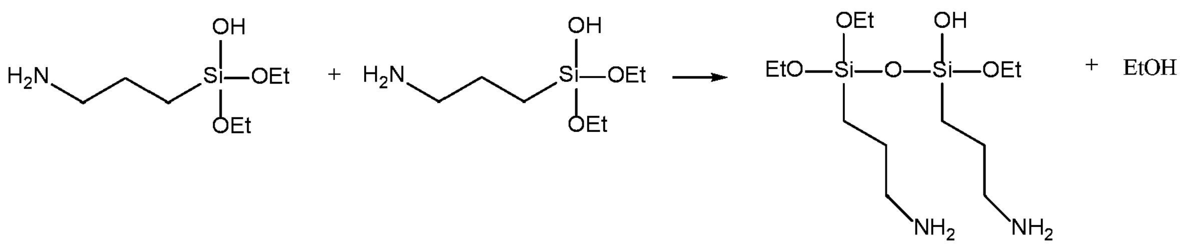

An der Kondensationsreaktion können sowohl partiell hydrolysierte als auch vollständig hydrolysierte $C_1$-$C_6$-Alkoxysilane teilnehmen, die eine Kondensation mit noch nicht abreagierten, partiell oder auch vollständig hydrolysierten $C_1$-$C_6$-Alkoxysilanen durchlaufen.

Mögliche Kondensationsreaktionen sind beispielsweise

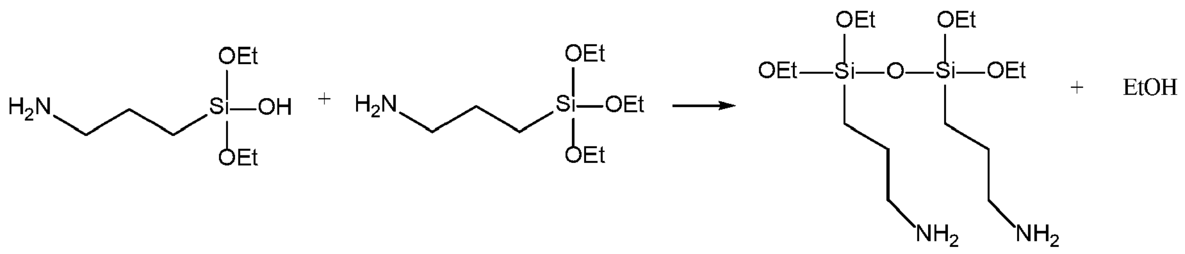

und/oder

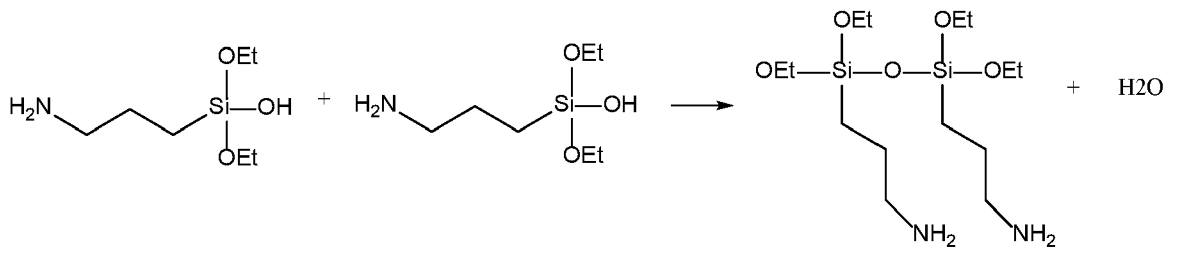

und/oder

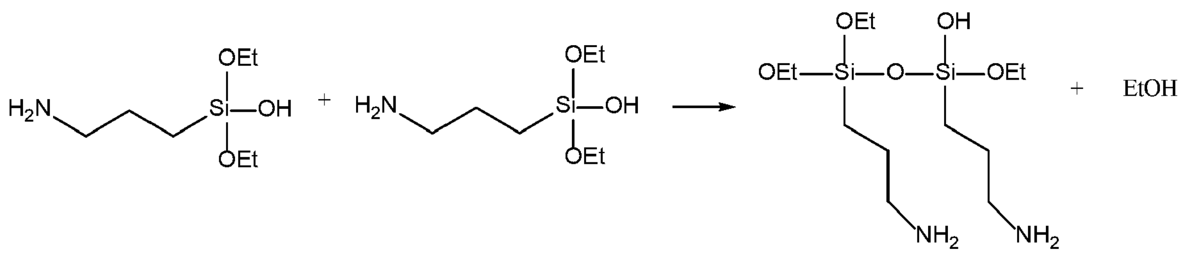

Mittel (B): Kondensation am Beispiel von Methyltrimethoxysilan

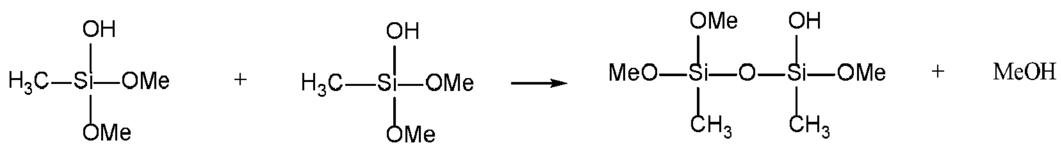

und/oder

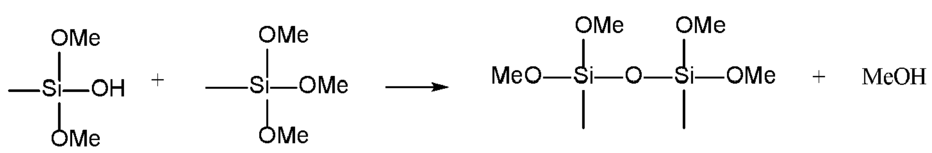

In den obigen beispielhaften Reaktionsschemata ist jeweils die Kondensation zu einem Dimer gezeigt, jedoch sind auch weitergehende Kondensationen zu Oligomeren mit mehreren Silan-Atomen möglich und auch bevorzugt, da sie zur der vorbeschriebenen Vernetzung des Siloxan-Gemisches führen.

<u>Reaktionsgefäße</u>

Die Herstellung der Mittel (A) und (B) aus organischen $C_1$-$C_6$-Alkoxysiloxanen erfolgt bevorzugt in einem hierfür geeigneten Reaktor oder Reaktionsgefäß. Ein für kleinere Ansätze sehr gut geeignetes Reaktionsgefäß ist beispielsweise ein üblicherweise für chemische Reaktionen eingesetzter Glaskolben mit einem Fassungsvermögen von 1 Liter, 3 Litern

oder 5 Litern handeln, beispielsweise ein 3-Liter Ein- oder Mehrhalskolben mit Schliffen.

Als Reaktor wird ein abgegrenzter Raum (Behältnis, Behälter) bezeichnet, der speziell dafür konstruiert und hergestellt wurde, um darin unter definierten Bedingungen bestimmte Reaktionen ablaufen lassen und steuern zu können.

Für größere Ansätze hat es sich als vorteilhaft herausgestellt, die Reaktion in Reaktoren aus Metall durchzuführen. Typische Reaktoren können beispielsweise eine Füllmenge von 10-Litern, 20-Litern oder 50-Litern umfassen. Größere Reaktoren für den Produktionsbereich können auch Füllmengen von 100-Litern, 500-Litern oder 1000-Litern umfassen.

Doppelwandreaktoren besitzen zwei Reaktor-Hüllen bzw. Reaktor-Wandungen, wobei in dem zwischen beiden Wandungen befindlichen Bereich eine Temperier-Flüssigkeit zirkulieren kann. Diese ermöglicht eine besonders gute Einstellung der Temperatur auf die benötigten Werte.

Als besonders gut geeignet hat sich auch der Einsatz von Reaktoren, insbesondere DoppelwandReaktoren mit vergrößerter Wärme-Austauschfläche erwiesen, wobei der Wärmeaustauch hierbei entweder durch interne Einbauten oder auch durch Einsatz eines externen Wärmeaustauschers erfolgen kann.

Entsprechende Reaktoren sind beispielsweise Laborreaktoren der Firma IKA. In diesem Zusammenhang können die Modelle "LR-2.ST" oder das Modell "magic plant" genannt werden.

Weitere einsetzbare Reaktoren sind Reaktoren mit Dünnfilm-Verdampfer, da auf diesem Wege eine sehr gute Wärmeabfuhr und damit eine besonders exakte Temperierung vorgenommen werden kann. Dünnfilm-Verdampfer werden alternativ auch als Dünnschicht-Verdampfer bezeichnet. Dünnschicht-Verdampfer können beispielsweise von der Asahi Glassplant Inc. kommerziell erworben werden.

Vermischen der Mittel (A) und (B)

Die erfindungsgemäße kosmetische Zusammensetzung wird nun durch Vermischen eines ersten Mittels (A) mit einem zweiten Mittel (B) erhalten.

Dieses Vermischen erfolgt erfindungsgemäß vor der Anwendung der kosmetischen Zusammensetzung auf dem Keratinmaterial. Hierbei kann das Vermischen der Mittel (A) und (B) entweder kurz vor der Anwendung erfolgen, oder aber die beiden Mittel (A) und (B) können beispielsweise zunächst separat produziert, dann für einen bestimmten Zeitraum gelagert und danach miteinander vermischt werden. Nach diesem Mischvorgang kann die kosmetische Zubereitung nochmals für einen bestimmten Zeitraum gelagert werden, bevor sie dann auf dem Keratinmaterial angewendet wird. Wie zuvor beschrieben kann auch eine nochmalige Hydrolyse-Reaktion mit der Mischung (A) + (B) durchgeführt werden.

Die Mittel (A) und (B) können zunächst beispielsweise für einige Tage, Wochen oder Monate gelagert werden, bevor sie miteinander vermischt werden. Auch die durch Vermischen der Mittel (A) und (B) hergestellte kosmetische Zubreitung kann nochmals für einige Zeit, beispielsweise eine Tage, Wochen oder Monate, gelagert werden.

Der Vernetzungsgrad in der erfindungsgemäßen kosmetischen Zusammensetzung hängt auch von dem Mischungsverhältnis ab, in dem die beiden Mittel (A) und (B) miteinander vermischt werden. Zur Erzeugung von besonders stabilen, resistenten und reproduzierbaren Filmen bzw. Coatings auf dem Keratinmaterial hat es sich als besonders bevorzugt erwiesen, die Mittel (A) und (B) in einem bestimmten Mengenverhältnis miteinander zu vermischen.

Besonders gute Ergebnisse konnten erhalten werden, wenn das Mittel (A) und das Mittel (B) in einem Gewichtsverhältnis (A)/(B) von 1:5 bis 5:1, bevorzugt von 1:4 bis 4:1, weiter bevorzugt von 1:3 bis 3:1 und ganz besonders bevorzugt von 1:2 bis 2:1 miteinander vermischt werden.

Im Rahmen einer besonders bevorzugten Ausführungsform ist ein erfindungsgemäße kosmetische Zusammensetzung dadurch gekennzeichnet, dass sie erhalten wird durch Vermischen des ersten Mittels (A) mit dem zweiten Mittel (B) in einem Gewichtsverhältnis (A)/(B) von 1:5 bis 5:1, bevorzugt von 1:4 bis 4:1, weiter bevorzugt von 1:3 bis 3:1 und ganz besonders bevorzugt von 1:2 bis 2:1.

Rechenbeispiel

Zunächst wurden die beiden Mittel (A) und (B) wie zuvor beschrieben hergestellt, in separate Container abgefüllt, und für einige Tage gelagert. Danach wurden 50 g des Mittels (A) mit 50 g des Mittels (B) vermischt.

Das Gewichtsverhältnis (A)/B) beträt 50/50 und liegt damit bei 1 : 1.

Diese Mischung aus (A) und (B) kann entweder nochmals separat abgefüllt und gelagert werden, oder aber die Mischung wird durch Zugabe einer weiteren Menge Wasser einer nochmaligen Hydrolyse-Reaktion unterworfen.

Verfahren zur Herstellung der kosmetischen Zusammensetzung

Das erfindungsgemäße Verfahren zur Herstellung der kosmetischen Zusammensetzung kann verschiedene Schritte umfassen.

Besonders bevorzugt ist ein erfindungsgemäßes Verfahren, umfassend die folgenden Schritte

(1) Bereitstellen eines oder mehrerer organischer $C_1$-$C_6$-Alkoxysilane (a1) in einem Reaktionsgefäß,
(2) Herstellung des Mittels (A) durch Vermischen der organischen $C_1$-$C_6$-Alkoxy-Silane (a1) mit Wasser (a2), gegebenenfalls unter Rühren und/oder Erhitzen des Gemisches im Reaktionsgefäß auf eine Temperatur von 30 bis 90 °C,
(3) Bereitstellen eines oder mehrerer organischer $C_1$-$C_6$-Alkoxysilane (b1) in einem Reaktionsgefäß, das von dem Reaktionsgefäß in Schritt (1) verschieden ist,
(4) Herstellung des Mittels (B) durch Abmischung der organischen $C_1$-$C_6$-Alkoxy-Silane (b1) mit Wasser (b2), gegebenenfalls unter Rühren und/oder Erhitzen des Gemisches im Reaktionsgefäß auf eine Temperatur von 30 bis 90 °C,
(5) Vermischen der Mittel (A) und (B), gegebenenfalls unter Rühren und/oder Erhitzen des Gemisches im Reaktionsgefäß auf eine Temperatur von 30 bis 90 °C.

Im Anschluss an das Vermischen der Mittel (A) und (B) in Schritt (5) kann das auf diese Weise erhaltene Gemisch entweder zur Lagerung oder für die Bereitstellung des Produktes abgefüllt werden, oder aber es schließt sich eine weitere Reaktion an, bei der mindestens eine Substanz aus der Gruppe aus Wasser, Alkalisierungsmittel(n), Säure(n) und Lösungsmittel(n) hinzugefügt wird.

Ganz besonders bevorzugt ist demzufolge ein Verfahren umfassend weiterhin das

(6) Hinzufügen von Wasser und/oder mindestens einem Alkalisierungsmittel und/oder mindestens einer Säure und/oder mindestens einem Lösungsmittel zu der Mischung der Mittel (A) und (B) und/oder das
(7) Abfüllen des in Schritt (5) bzw. in Schritt (6) erhaltenen Gemisches aus dem Reaktionsgefäß.

Besonders gut geeignete Alkalisierungmsittel, die alternativ auch als Basen bezeichnet werden können, sind die, die bereits zuvor beschrieben wurden.

Besonders gut geeignete Säuren sind die, die bereits zuvor beschrieben wurden.

Besonders gut geeignete Lösungsmittel sind die, die bereits zuvor beschrieben wurden.

Im Rahmen einer weiteren ganz besonders bevorzugten Ausführungsform ist ein erfindungsgemäßes Verfahren dadurch gekennzeichnet, dass das Alkalisierungsmittel ausgewählt ist aus der Gruppe aus Natriumhydroxid, Kaliumhydroxid, Litiumhydroxid, Magnesiumhydroxid und Calciumhydroxid.

Im Rahmen einer weiteren ganz besonders bevorzugten Ausführungsform ist ein erfindungsgemäßes Verfahren dadurch gekennzeichnet dass das Lösungsmittel ausgewählt ist aus der Gruppe aus Poly-$C_1$-$C_6$-Alkylenglycolen, 1,2-Propylenglycol, 1,3-Propylenglycol, 1,2-Butylenglycol, Dipropylenglycol, Ethanol, Isopropanol, Diethylenglycolmonoethylether, Glycerin, Phenoxyethanol und Benzylalkohol.

Weiterhin besonders gut geeignet ist auch ein Verfahren, umfassend die folgenden Schritte

(1) Bereitstellen eines oder mehrerer organischer $C_1$-$C_6$-Alkoxysilane (a1) in einem Reaktionsgefäß,
(2) Herstellung des Mittels (A) durch Vermischen der organischen $C_1$-$C_6$-Alkoxy-Silane (a1) mit Wasser (a2), gegebenenfalls unter Rühren und/oder Erhitzen des Gemisches im Reaktionsgefäß auf eine Temperatur von 30 bis 90 °C,
(3) Bereitstellen eines oder mehrerer organischer $C_1$-$C_6$-Alkoxysilane (b1) in einem Reaktionsgefäß, das von dem Reaktionsgefäß in Schritt (1) verschieden ist,
(4) Vermischen eines oder mehrerer Katalysatoren (b3) mit Wasser (b2),
(5) Herstellung des Mittels (B) durch Abmischung der organischen $C_1$-$C_6$-Alkoxy-Silane (b1) mit dem Gemisch aus Katalysator (b3) und Wasser (b2), gegebenenfalls unter Rühren und/oder Erhitzen des Gemisches im Reaktionsgefäß auf eine Temperatur von 30 bis 90 °C,
(6) Vermischen der Mittel (A) und (B), gegebenenfalls unter Rühren und/oder Erhitzen des Gemisches im Reaktionsgefäß auf eine Temperatur von 30 bis 90 °C.

In bei dieser Ausführungsformenkann sich an das Vermischen der Mittel (A) und (B), das gegebenenfalls unter Rühren und/oder Erhitzen des Gemisches im Reaktionsgefäß auf eine Temperatur von 30 bis 90 °C erfolt, kann sich auch noch ein weiterer Reaktionsschritt anschließen.

Ganz besonders bevorzugt ist demzufolge ein Verfahren umfassend weiterhin das

(7) Hinzufügen von Wasser und/oder mindestens einem Alkalisierungsmittel und/oder mindestens einer Säure und/oder mindestens einem Lösungsmittel zu der Mischung der Mittel (A) und (B) und/oder das
(8) Abfüllen des in Schritt (6) bzw. in Schritt (7) erhaltenen Gemisches aus dem Reaktionsgefäß.

Weiterhin geeignet ist auch ein Verfahren, umfassend die folgenden Schritte

(1) Bereitstellen eines oder mehrerer organischer $C_1$-$C_6$-Alkoxysilane (a1) in einem Reaktionsgefäß,
(2) Vermischen eines oder mehrerer Katalysatoren (a3) mit Wasser (a2),
(3) Herstellung des Mittels (A) durch Abmischung der organischen $C_1$-$C_6$-Alkoxy-Silane (a1) mit dem Gemisch aus Katalysator (a3) und Wasser (a2), gegebenenfalls unter Rühren und/oder Erhitzen des Gemisches im Reaktionsgefäß auf eine Temperatur von 30 bis 90 °C,
(4) Bereitstellen eines oder mehrerer organischer $C_1$-$C_6$-Alkoxysilane (b1) in einem Reaktionsgefäß, das von dem Reaktionsgefäß in Schritt (1) verschieden ist,
(5) Vermischen eines oder mehrerer Katalysatoren (b3) mit Wasser (b2),
(6) Herstellung des Mittels (B) durch Abmischung der organischen $C_1$-$C_6$-Alkoxy-Silane (b1) mit dem Gemisch aus Katalysator (b3) und Wasser (b2), gegebenenfalls unter Rühren und/oder Erhitzen des Gemisches im Reaktionsgefäß auf eine Temperatur von 30 bis 90 °C,
(7) Vermischen der Mittel (A) und (B), gegebenenfalls unter Rühren und/oder Erhitzen des Gemisches im Reaktionsgefäß auf eine Temperatur von 30 bis 90 °C.

In bei dieser Ausführungsformenkann sich an das Vermischen der Mittel (A) und (B), das gegebenenfalls unter Rühren und/oder Erhitzen des Gemisches im Reaktionsgefäß auf eine Temperatur von 30 bis 90 °C erfolt, kann sich auch noch ein weiterer Reaktionsschritt anschließen.

Ganz besonders bevorzugt ist demzufolge ein Verfahren umfassend weiterhin das

(8) Hinzufügen von Wasser und/oder einem Alkalisierungsmittel und/oder einer Säure und/oder mindestens einem Lösungsmittel zu der Mischung der Mittel (A) und (B) und/oder das
(9) Abfüllen des in Schritt (7) bzw. in Schritt (8) erhaltenen Gemisches aus dem Reaktionsgefäß.

Kosmetische Zusammensetzung zur Behandlung von keratinischem Material

Die kosmetischen Zusammensetzungen, die über das Verfahren des ersten Erfindugsgegenstands hergestellt wurden, eignen sich besonders gut zur Behandlung von keratinischem Material.

Ein zweiter Gegenstand der vorliegenden Erfindung ist daher eine kosmetische Zusammensetzung zur Behandlung von keratinischem Material, insbesondere menschlichen Haaren, die nach einem Verfahren hergestellt wurde, wie es bei der Beschreibung des ersten Erfindungsgegenstands im Detail offenbart wurde.

Verwendung der kosmetischen Zusammensetzung zur Behandlung von keratinischem Material

Die kosmetische Zusammensetzung, die über das Verfahren des ersten Erfindugsgegenstands hergestellt wurde, eignet sich besonders gut zur zur Behandlung keratinischem Material, insbesondere zur Färbung von keratinischem Material, insbesondere zur Färbung von menschlichen Haaren.

Ein dritter Gegenstand der vorliegenden Erfindungist daher die Verwendung einer kosmetischen Zusammensetzung, die nach einem Verfahren hergestellt wurde, wie es bei der Beschreibung des ersten Erfindungsgegenstands im Detail offenbart wurde, zur Behandlung keratinischem Material, insbesondere zur Färbung von keratinischem Material, insbesondere zur Färbung von menschlichen Haaren.

Betreffend die weiteren bevorzugten Ausführungsformen der erfindungsgemäßen kosmetischen Zusammensetzung und und ihrer Verwendung gilt *mutatis mutandis* das zu dem erfindungsgemäßen Verfahren gesagte.

**Patentansprüche**

**1.** Verfahren zur Herstellung einer kosmetischen Zusammensetzung zur Behandlung von keratinischem Material, insbesondere menschlichen Haaren, die erhalten wird durch Vermischen von einem ersten Mittel (A) mit einem zweiten Mittel (B), wobei

- das erste Mittel (A) erhalten wird durch Reaktion von einem oder mehreren organischen $C_1$-$C_6$-Alkoxysilanen (a1) mit Wasser (a2),
wobei das oder die organischen $C_1$-$C_6$-Alkoxysilane (a1) ausgewählt sind aus der Gruppe aus (3-Aminopropyl)triethoxysilan, (3-Aminopropyl)trimethoxysilan, (2-Aminoethyl)-triethoxysilan, (2-Aminoethyl)trimethoxysilan, (3-Dimethylaminopropyl)triethoxysilan, (3-Dimethylaminopropyl)tri-methoxysilan, (2-Dimethylaminoethyl)triethoxysilan und (2-Dimethyl-aminoethyl)-trimethoxysilan, und

- das zweite Mittel (B) erhalten wird durch Reaktion von einem oder mehreren organischen $C_1$-$C_6$-Alkoxysilanen (b1) mit Wasser (b2),

wobei das oder die organischen $C_1$-$C_6$-Alkoxysilane (b1) ausgewählt sind aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Vinyltrimethoxysilan, Vinyltriethoxysilan Tetramethoxysilan und Tetraethoxysilan,
**dadurch gekennzeichnet, dass**

- das Mittel (A) keine organischen $C_1$-$C_6$-Alkoxysilane aus der Gruppe (b1) enthält und
- das Mittel (B) keine organischen $C_1$-$C_6$-Alkoxysilane aus der Gruppe (a1) enthält.

2. Verfahren nach Anspruch 1, **dadurch gekennzeichnet, dass** die organischen $C_1$-$C_6$-Alkoxysilane (a1) im Mittel (A) mit einer Wassermenge (a2) zur Reaktion gebracht werden, die der Molmenge an Wasser entspricht, die sich nach der Gleichung (G-1) bestimmt,

$$X = [(n_I(\text{Alkoxysilane (a1)}) \times n_{II}(\text{Alkoxygruppen})] / n(H_2O\ (a2)) \qquad \text{(G-1)}$$

wobei

$n(H_2O)$ die Molmenge des im Mittel (A) eingesetzten Wassers (a2) ist,
$n_I$ die Molmenge der im Mittel (A) eingesetzten organischen $C_1$-$C_6$-Alkoxysilane (a1) ist,
$n_{II}$, die Anzahl der $C_1$-$C_6$-Alkoxygruppenpro im Mittel (A) eingesetzten organischen $C_1$-$C_6$-Alkoxysilan (a1) ist, und
X eine Zahl von 0,1 bis 100,0, bevorzugt von 1,0 bis 50,0 weiter bevorzugt von 1,2 bis 30,0, nochweiter bevorzugt von 1,3 bis 10 und ganz besonders bevorzugt von 1,8 bis 4,5 darstellt.

3. Verfahren nach einem der Ansprüche 1 bis 2, **dadurch gekennzeichnet, dass** die Gesamtmenge der im Mittel (A) eingesetzten Bestandteile (a1) und (a2) einen Gewichtsanteil von mindestens 60 Gew.-%, bevorzugt von mindestens 70 Gew.-%, weiter bevorzugt von mindestens 80 Gew.-%, noch weiter bevorzugt von mindestens 90 Gew.-% und ganz besonders bevorzugt von mindestens 98 Gew.-%, bezogen auf das Gesamtgewicht des Mittels (A), ausmacht.

4. Verfahren nach einem der Ansprüche 1 bis 3, **dadurch gekennzeichnet**, die organischen $C_1$-$C_6$-Alkoxysilane (b1) im Mittel (B) mit einer Wassermenge (b2) zur Reaktion gebracht werden, die der Molmenge an Wasser entspricht, die sich nach der Gleichung (G-2) bestimmt,

$$Y = [(m_I(\text{Alkoxysilane (b1)}) \times m_{II}(\text{Alkoxygruppen})] / m(H_2O\ (b2)) \qquad \text{(G-2)}$$

wobei

$m(H_2O)$ die Molmenge des im Mittel (B) eingesetzten Wassers (b2) ist,
$M_I$ die Molmenge der im Mittel (B) eingesetzten organischen $C_1$-$C_6$-Alkoxysilane (b1) ist,
$M_{II}$ die Anzahl der $C_1$-$C_6$-Alkoxygruppenpro im Mittel (B) eingesetzten organischen $C_1$-$C_6$-Alkoxysilan (b1) ist, und
Y eine Zahl von 0,1 bis 100,0, bevorzugt von 1,0 bis 50,0 weiter bevorzugt von 1,2 bis 30,0, nochweiter bevorzugt von 1,3 bis 10 und ganz besonders bevorzugt von 1,8 bis 4,5 darstellt.

5. Verfahren nach einem der Ansprüche 1 bis 4, **dadurch gekennzeichnet, dass** das zweite Mittel (B) erhalten wird durch Reaktion von einem oder mehreren organischen $C_1$-$C_6$-Alkoxysilanen (b1) mit Wasser (b2) und mindestens einem Katalysator (b3),

6. Verfahren nach Anspruch 5, **dadurch gekennzeichnet, dass** der Katalysator (b3) ausgewählt ist aus der Gruppe aus anorganischen und organischen Säuren, bevorzugt aus der Gruppe aus Schwefelsäure, Salzsäure, Phosphorsäure, Maleinsäure, Zitronensäure, Weinsäure, Äpfelsäure, Milchsäure, Essigsäure, Methan-sulfonsäure, Benzoesäure, Malonsäure, Oxalsäure und 1-Hydroxyethan-1,1-diphosphonsäure.

**7.** Verfahren nach einem der Ansprüche 1 bis 6, **dadurch gekennzeichnet, dass** die Gesamtmenge der im Mittel (B) eingesetzten Bestandteile (b1) und (b2) einen Gewichtsanteil von mindestens 60 Gew.-%, bevorzugt von mindestens 70 Gew.-%, weiter bevorzugt von mindestens 80 Gew.-%, noch weiter bevorzugt von mindestens 90 Gew.-% und ganz besonders bevorzugt von mindestens 98 Gew.-%, bezogen auf das Gesamtgewicht des Mittels (B), ausmacht.

**8.** Verfahren nach einem der Ansprüche 1 bis 7, umfassend die folgenden Schritte

(1) Bereitstellen eines oder mehrerer organischer $C_1$-$C_6$-Alkoxysilane (a1) in einem Reaktionsgefäß,
(2) Herstellung des Mittels (A) durch Vermischen der organischen $C_1$-$C_6$-Alkoxy-Silane (a1) mit Wasser (a2), gegebenenfalls unter Rühren und/oder Erhitzen des Gemisches im Reaktionsgefäß auf eine Temperatur von 30 bis 90 °C,
(3) Bereitstellen eines oder mehrerer organischer $C_1$-$C_6$-Alkoxysilane (b1) in einem Reaktionsgefäß, das von dem Reaktionsgefäß in Schritt (1) verschieden ist,
(4) Herstellung des Mittels (B) durch Abmischung der organischen $C_1$-$C_6$-Alkoxy-Silane (b1) mit Wasser (b2), gegebenenfalls unter Rühren und/oder Erhitzen des Gemisches im Reaktionsgefäß auf eine Temperatur von 30 bis 90 °C,
(5) Vermischen der Mittel (A) und (B), gegebenenfalls unter Rühren und/oder Erhitzen des Gemisches im Reaktionsgefäß auf eine Temperatur von 30 bis 90 °C.

**9.** Verfahren nach Anspruch 8, umfassend weiterhin das

(6) Hinzufügen von Wasser und/oder mindestens einem Alkalisierungsmittel und/oder mindestens einer Säure und/oder mindestens einem Lösungsmittel zu der Mischung der Mittel (A) und (B), und/oder das
(7) Abfüllen des in Schritt (5) bzw. in Schritt (6) erhaltenen Gemisches aus dem Reaktionsgefäß.

**10.** Verfahren nach Anspruch 9, **dadurch gekennzeichnet, dass** das Alkalisierungsmittel ausgewählt ist aus der Gruppe aus Natriumhydroxid, Kaliumhydroxid, Litiumhydroxid, Magnesiumhydroxid und Calciumhydroxid.

**11.** Verfahren nach einem der Ansprüche 9 bis 10, **dadurch gekennzeichnet, dass** das Lösungsmittel ausgewählt ist aus der Gruppe aus Poly-$C_1$-$C_6$-Alkylenglycolen, 1,2-Propylenglycol, 1,3-Propylenglycol, 1,2-Butylenglycol, Dipropylenglycol, Ethanol, Isopropanol, Diethylenglycolmonoethylether, Glycerin, Phenoxyethanol und Benzylalkohol.

**12.** Kosmetische Zusammensetzung zur Behandlung von keratinischem Material, insbesondere menschlichen Haaren, die nach einem Verfahren gemäß einem der Ansprüche 1 bis 11 hergestellt wurde.

**13.** Verwendung einer kosmetischen Zusammensetzung, die nach einem Verfahren gemäß einem der Ansprüche 1 bis 11 hergestellt wurde, zur Behandlung keratinischem Material, insbesondere zur Färbung von keratinischem Material, insbesondere zur Färbung von menschlichen Haaren.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2168633 B1
- WO 2013068979 A2


### In der Beschreibung aufgeführte Nicht-Patentliteratur


- *Journal of Organometallic Chemistry,* 2001, vol. 625, 208-216